(19) Europäisches Patentamt
European Patent Office
Office européen des brevets

(11) Veröffentlichungsnummer: **0 358 118 B1**

(12) **EUROPÄISCHE PATENTSCHRIFT**

(45) Veröffentlichungstag der Patentschrift: **16.03.94**

(51) Int. Cl.[5]: **C07C 233/66**, A61K 31/165, C07C 233/73, C07C 233/76, C07C 233/80, C07C 233/87

(21) Anmeldenummer: **89116153.1**

(22) Anmeldetag: **01.09.89**

Die Akte enthält technische Angaben, die nach dem Eingang der Anmeldung eingereicht wurden und die nicht in dieser Patentschrift enthalten sind.

# (54) Phenylamide - Verfahren zu ihrer Herstellung sowie diese Verbindungen enthaltende Arzneimittel.

(30) Priorität: **03.09.88 DE 3830054**

(43) Veröffentlichungstag der Anmeldung:
**14.03.90 Patentblatt 90/11**

(45) Bekanntmachung des Hinweises auf die Patenterteilung:
**16.03.94 Patentblatt 94/11**

(84) Benannte Vertragsstaaten:
**AT BE CH DE ES FR GB GR IT LI LU NL SE**

(56) Entgegenhaltungen:
**EP-A- 0 008 391**
**EP-A- 0 148 725**
**EP-A- 0 268 267**
**FR-A- 2 376 858**
**GB-A- 2 164 648**
**EP-A- 0 118 752**
**EP-A- 0 170 105**
**DE-A- 2 323 022**
**GB-A- 2 038 814**
**US-A- 4 008 274**

**PATENT ABSTRACTS OF JAPAN, Band 5, Nr. 79 (C-56)(751), 23. Mai 1981; & JP-A-56 29 552**

**CHEMICAL ABSTRACTS, Band 94, Nr. 4, 26. Januar 1981, Seite 492, Spalte 2, Zusammenfassung Nr. 22 881f, Columbus, Ohio, USA; & JP-A-8006321**

(73) Patentinhaber: **BOEHRINGER MANNHEIM GMBH**

**D-68298 Mannheim(DE)**

(72) Erfinder: **von der Saal, Wolfgang, Dr. rer. nat.**
**Neuer Burgweg 3**
**D-6940 Weinheim(DE)**
Erfinder: **Mertens, Alfred, Dr. rer. nat.**
**Beethovenstrasse 20**
**D-6905 Schriesheim(DE)**
Erfinder: **Zilch, Harald, Dr. rer. nat.**
**Alsenweg 2 A**
**D-6800 Mannheim 31(DE)**
Erfinder: **Boehm, Erwin, Dr. med.**
**Hinterer Rindweg 37**
**D-6802 Ladenburg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**CHEMICAL ABSTRACTS, Band 73, Nr. 15, 12.Oktober 1970, Seite 333, Spalte 1, Zusammenfassung Nr. 76 840b, Columbus, Ohio, USA; & SU-A-6 805 360**

**PATENT ABSTRACTS OF JAPAN, Band 4, Nr. 166 (C-31)(648), 18. November 1980; & JP-A-55 108 846**

**PATENT ABSTRACTS OF JAPAN, Band 4, Nr. 159 (C-30)(641), 6. November 1980; & JP-A-55 105 655**

## Beschreibung

Die vorliegende Erfindung betrifft Arzneimittel enthaltend Phenylamide der allgemeinen Formel I,

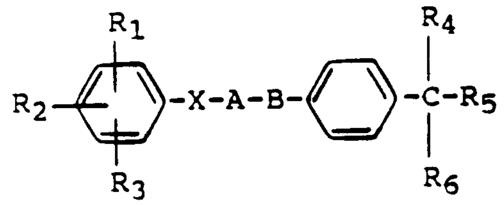

(I)

in der

$R_1$, $R_2$, $R_3$, gleich oder verschieden sein können und jeweils ein Wasserstoffatom, eine Alkyl-, Alkenyl-, Alkinyl-, Cycloalkyl-, Cycloalkenyl-, Phenyl-, Halogen-, Nitro-, Amino-, Formyl-, Hydrory-, Mercapto-, Cyanogruppe, eine durch eine Alkyl-, Alkenyl-, Alkinyl-, Cycloalkyl-, Phenyl-, Benzyl-, Pyridinyl-, Alkylsulfonyl-, Trifluormethylsulfonyl-, Alkylcarbonyl-, Cyanalkyl-, Hydroryalkyl-, Dialkylaminoalkyl-, Aminocarbonylalkyl-, Dialkylaminocarbonylalkyl-, N-Cycloalkyl-N-alkylaminocarbonylalkyl-, Carboxyalkyl-, Alkoxycarbonylalkyl- oder Alkoxyalkylgruppe substituierte Hydroxygruppe, eine durch eine oder zwei Trifluormethylsulfonyl-, Alkylcarbonyl-, Formyl-, Aminocarbonyl-, Alkylaminocarbonyl- oder Alkylgruppen substituierte Aminogruppe, eine durch eine Alkylgruppe substituierte Carbonylgruppe, eine durch eine Amino-, Alkylamino-, Piperidino-, Morpholino- oder Thiomorpholinogruppe substituierte Sulfonylgruppe, eine Alkylthio-, Alkylsulfinyl- oder Alkylsulfonylgruppe bedeuten können, oder in der zwei zueinander ortho-ständige Substituenten $R_2$ und $R_3$ zusammen mit den C-Atomen, an die sie gebunden sind, einen fünf- oder sechsgliedrigen Ring bilden,

X eine Bindung, eine Alkylengruppe, oder für den Fall, daß A die Carbonylgruppe -CO- bedeutet, auch die Alkenylengruppe bedeuten kann, und

A und B verschieden sind und die Carbonylgruppe -CO- oder die Iminogruppe -NH- bedeuten, und

$R_4$ eine Methyl-, Cyano-, Aminocarbonyl- oder Aminomethylgruppe bedeutet,

$R_5$ ein Wasserstoffatom, oder eine Alkylgruppe bedeutet,

$R_6$ eine Alkyl- oder Cycloalkylgruppe bedeutet,

oder

$R_5$ und $R_6$ zusammen mit dem C-Atom, an das sie gebunden sind, einen Cycloalkylring bedeuten,

deren optisch aktiven Formen, deren Tautomere und deren physiologisch verträglichen Salze anorganischer und organischer Säuren. Ferner bezieht sich die vorliegende Erfindung auf neue Phenylamide, Verfahren zur Herstellung dieser Verbindungen sowie deren Verwendung zur Herstellung von Arzneimitteln, insbesondere von Arzneimitteln mit erythrozytenaggregationshemmender Wirkung.

Für den Fall, daß Verbindungen der allgemeinen Formel I ein Asymmetriezentrum oder eine Asymmetrieebene besitzen, sind auch die optisch aktiven Formen und racemischen Gemische dieser Verbindungen Gegenstand der Erfindung.

Arzneimittel, die diese Verbindungen enthalten, weisen wertvolle pharmakologische Eigenschaften auf, insbesondere wirken sie hemmend auf die Erythrozytenaggregation und können somit Verwendung finden zur Behandlung von Krankheiten, bei denen in der Pathogenese die Erythrozytenaggregation eine wichtige Rolle spielt, wie z.B. periphere, coronare und cerebrale Durchblutungsstörungen, Schockzustände, usw. Die Verbindungen beeinflussen darüberhinaus die Thrombozytenfunktion, steigern die Herzkraft und wirken blutdrucksenkend.

Einige strukturell nahestehenden Verbindungen, die von der vorliegenden Erfindung nicht umfaßt werden, sind als Arzneimittel bereits bekannt.

So beschreiben die Patentanmeldungen CH 609,558 bzw. US 398522 (18. Sept. 1973, L.Givaudan et Cie.) die antibakterielle Wirkung von Verbindungen, in denen $R_4$, $R_5$, $R_6$ eine Methylgruppe, B eine Carbonyl-, A eine Iminogruppe -NH-, X einen Valenzstrich und einer der Substituenten $R_1$, $R_2$, $R_3$ in meta-Position des Phenylrings die Trifluormethylgruppe und die beiden anderen ein Wasserstoff- oder Halogenatom oder eine Trifluormethylgruppe darstellen, insbesondere die Verbindungen 4-(1,1-Dimethyl-ethyl)-N-[3-trifluormethyl)phenyl]benzamid und von N-[4-Chlor-3-trifluormethyl)phenyl)-4-(1,1-dimethylethyl)benzamid.

Die Patentanmeldung US 4160097 (Westwood Pharmaceuticals, 3. Juli 1979) beschreibt die entzündungshemmende Wirkung von 4-(1,1-Dimethylethyl)-N-[2-(1H-imidazol-1-yl)phenyl]benzamid.

Von den folgenden Verbindungen, die von der vorliegenden Erfindung umfaßt werden, ist die Synthese beschrieben, jedoch nicht ihre Verwendung in Arzneimitteln:

N-[2-(Aminocarbonyl)phenyl]-4-(1,1-dimethylethyl)benzamid in der Patentanmeldung US 450870 (16.3.76, FMC Corp.);

4-(1,1-Dimethylethyl)-N-phenyl-benzamid in S.Ito et al., Nippon Noyaku Gakkashi 10, 697;

N-(2,6-Dihydroxyphenyl)-4-(1,1-dimethylethyl)benzamid in der Patentanmeldung GB 2018453 ( 14. Okt. 1979, Eastman Kodak);

4-(1,1-Dimethylethyl)-N-[2-(1-methylethoxy)phenyl]benzamid in der Patentanmeldung JP 77/496 (6. Jan. 1977), Kumiai Chemical Industry Co);

4-(1,1-Dimethylethyl)-N-[3-(1,1-dimethylethyl)-4-(octyloxy)phenyl]benzamid in der Patentanmeldung JP 55/6321 (17.1.1980, Konishiroku Photo Industry Co.);

2-[[4-(1,1-Dimethylethyl)benzoyl]amino]benzoesäure-methylester in K. Osann et al., Agric.Biol.Chem. 44, 2143;

N-(2,3-Dichlorphenyl)-4-(1,1-dimethylethyl)-benzamid in der Patentanmeldung JP 55/76851 (5. Okt. 1978, Hodogaya Chemical Co);

4-(1,1-Dimethylethyl)-N-[4-(1-methylethoxy)phenyl]benzamid in der Patentanmeldung JP 55/108846 (21.6.1980, Kumiai Chemical Ind.);

4-(1,1-Dimethylethyl)-N-[4-(1,1-dimethylethyl)phenyl]benzamid in F. Bell, J.A.Gibson, R.D. Wilson, J. Chem. Soc. 1956, 2335;

4-(1,1-Dimethylethyl)-N-(3-ethyl-4-hydroxyphenyl)benzamid in der Patentanmeldung ZA 68/5360 (19. Feb. 1970, ICI);

4-(1,1-Dimethylethyl)-N-[3-(1,1-dimethylethyl)-4-hydroxy-5-methylphenyl]benzamid in der Patentanmeldung US 4025487 (24. Mai 1977, Ciba-Geigy);

4-(1,1-Dimethylethyl)-N-(2-hydroxy-4-nitrophenyl)benzamid, N-(4-Amino-2-hydroxyphenyl)-4-(1,1-dimethylethyl)benzamid und 4-(1,1-Dimethylethyl)-N-[2-hydroxy-4-[(2-methyl-1-oxo-2-propenyl)amino]phenyl]-benzamid in der Patentanmeldung DE 2156480 (6. Juni 1972, Agfa);

N-(5-Chlor-2-hydroxy-4-nitrophenyl)-4-(1,1-dimethylethyl)benzamidund N-(4-Amino-5-chlor-2-hydroxyphenyl)-4-(1,1-dimethylethyl)benzamid in der Patentanmeldung JP 59/146050 21. August 1984, Fuji);

N-[4-(1,1-Dimethylethyl)phenyl]benzamid in R.M. Acheson, C.W.C. Harvey, J. Chem. Soc., Perkin I, 1976, 465.

N-[4-(1,1-Dimethylethyl)phenyl]-4-methyl-benzamid in der Patentanmeldung JP 58/187 391 (1. Nov. 1983, Fuji).

3-[[4-(1,1-dimethylethyl)benzoyl]amino]-4-methyl-benzoesäuremethylester und 4-Amino-3-[[4-(1,1-dimethylethyl)benzoyl]amino]benzoesäuremethylester in der Patentanmeldung BE 903254 (18. März 1986, C.I.R.D.).

Die "Alkyl-", "Alkenyl-" oder "Alkinyl-"teile der bei den Substituenten $R_1$, $R_2$, $R_3$, $R_5$ und $R_6$ genannten Gruppen können 1-6 bzw. 2-6 Kohlenstoffatome enthalten und geradkettig oder verzweigt sein. Bevorzugt kommen geradkettige Derivate mit 1-4 bzw. 2-4 Kohlenstoffatome in Frage.

Beispielsweise versteht man darunter die Methyl-, Ethyl-, n-Propyl-, i-Propyl-, n-Butyl-, i-Butyl-, tert.-Butyl-, Pentyl- und Hexylgruppe, die Methoxy-, Ethoxy-, n-Propyloxy-, i-Propyloxy-, Allyloxy-, i-Butenyloxy-, Propinyloxy-, n-Butyloxy-, tert.-Butyloxy-, i-Butyloxy-, n-Pentyloxy-, n-Hexyloxy-, Methylsulfonyloxy-, Ethylsulfonyloxy-, n-Propylsulfonyloxy-, i-Propylsulfonyloxy-, Methylcarbonyloxy-, Ethylcarbonyloxy-, Propylcarbonyloxy-, Cyanethyloxy-, Carboxyethoxy-, Ethoxycarbonylethoxy-, Methoxycarbonylethoxy-, Methoxyethyloxy-, Dimethylamino-, Diethylamino-, Acetylamino-, Propionylamino-, Methylsulfonylamino-, Ethylsulfonylamino-, n-Propylsulfonylamino-, i-Propylsulfonylamino-, Acetylaminocarbonylamino-, Propionylaminocarbonylamino-, Acetyl-, Propionyl-, Ethoxycarbonyl-, Propyloxycarbonyl-, Butyloxycarbonyl-, Methylaminocarbonyl-, Ethylaminocarbonyl-, Propylaminocarbonyl-, Dimethylaminocarbonyl-, Diethylaminocarbonyl-, Methylaminosulfonyl-, Ethylaminosulfonyl-, Methylthio-, Ethylthio-, Propylthio-, Methylsulfinyl-, Ethylsulfinyl-, Propylsulfinyl-, Methylsulfonyl-, Ethylsulfonyl- oder Propylsulfonylgruppe.

Weitere Substituenten $R_1$, $R_2$, $R_3$, die bevorzugt sind, sind die Phenylgruppe, für den Fall, daß $R_1$, $R_2$, $R_3$ eine substituierte Hydroxygruppe darstellt, die Phenyloxy-, Pyridinyloxy- und die Benzyloxygruppe; weiterhin Halogenatome wie Fluor, Chlor oder Brom, die Nitro-, Amino-, Formyl-, Hydroxy-, Mercapto-, Cyano-, Formylamino-, Carboxy-, Piperidinosulfonyl-, Morpholinosulfonyl- und die Thiomorpholinosulfonylgruppe und das Wasserstoffatom.

Bilden ortho-ständige Substituenten $R_1$ und $R_2$ zusammen mit den C-Atomen, an die sie gebunden sind einen fünf- oder sechsgliedrigen Ring, so resultieren daraus Bicyclen, unter denen man die Methylendioxyphenyl, die Ethylendioxyphenyl- und die Tetrahydronaphthylgruppe versteht.

Insbesondere sind bevorzugt für

$R_1$ das Wasserstoffatom, die Methyl-, Ethyl-, n-Propyl-, i-Propyl-, n-Butyl-, tert.-Butyl-, Fluor-, Chlor-, Brom-, Nitro-, Amino-, Hydroxy-, Mercapto-, Formyl-, Cyano-, Formylamino-, $C_1$-$C_4$-Alkyl, $C_1$-$C_4$-Alkoxy-, Allyloxy-, i-Butenyloxy-, Propargyloxy-, Cyanmethyloxy-, Ethoxycarbonylmethoxy-, $C_1$-$C_3$-Dialkylamino-, $C_1$-$C_3$-Alkylmercapto-, $C_1$-$C_3$-Alkylsulfinyl-, $C_1$-$C_3$-Alkylsulfonyl-, $C_1$-$C_3$-Alkylsulfonyloxy-, die Phenyl-, Phenyloxy-, Pyridinyloxy- und die Benzyloxygruppe,

für $R_2$ das Wasserstoffatom, eine $C_1$-$C_4$-Alkyl-, $C_1$-$C_4$-Alkoxy-, $C_1$-$C_3$-Dialkylamino-, Fluor-, Chlor-, Brom- und die Hydroxygruppe,

für $R_3$ das Wasserstoffatom, eine Hydroxy- und Methoxygruppe.

Bevorzugte monosubstituierte Phenyle sind Fluor-, Chlor-, Brom-, Hydroxy-, Amino-, Cyano-, $C_1$-$C_4$-Alkyl-, $C_1$-$C_4$-Alkoxy-, $C_2$-$C_4$-Alkenyloxy-, Propargyloxy-, Cyanmethoxy-, Methoxycarbonylmethyloxy-, Nitro-, $C_1$-$C_3$-Dialkylamino-, $C_1$-$C_3$-Alkylmercapto-, $C_1$-$C_3$-Alkylsulfinyl-, $C_1$-$C_3$-Alkylsulfonyl-, $C_1$-$C_3$-Alkylsulfonyloxy-, Trifluormethylsulfonyloxy- wobei der Substituent in 2-, 3- oder 4-Stellung stehen kann.

Bevorzugte disubstituierte Phenyle enthalten als Substituenten $C_1$-$C_4$-Alkyl-, Chlor-, Hydroxy-, $C_1$-$C_4$-Alkoxy-, Methylendioxy-, $C_1$-$C_4$-Alkylsulfonyloxy-, Trifluormethylsulfonyloxy-, $C_1$-$C_4$-Alkythio-, $C_1$-$C_4$-Alkylsulfinyl-, C1-C4-Alkylsulfonyl-, Trifluormethylsulfonylamino-, Cyanmethyloxy- oder Methoxycarbonylmethyloxygruppe, wobei die Gruppen gleich oder verschieden sein können und in 2,3-, 2,4-, 2,5-, 2,6-, 3,4- und 3,5-Stellung stehen können.

Bevorzugte trisubstituierte Phenyle weisen als Substituenten $C_1$-$C_4$-Alkyl-, $C_1$-$C_4$-Alkenyl-, $C_1$-$C_4$-Alkenyloxy- und Chlorgruppen auf, wobei die Reste gleich oder verschieden sein können und in 2,3,4-, 2,3,5-, 2,3,6-, 2,4,5-, 2,4,6- oder 3,4,5-Stellung stehen können.

Bedeutet in der allgemeinen Formel I X eine Alkylengruppe, so sind darunter die Methylen-, Ethylen-, Propylen- und die Butylengruppe zu verstehen. Als Alkenylengruppe kommt insbesondere die Vinylengruppe in Frage.

Bedeutet in der allgemeinen Formel I $R_5$ oder $R_6$ eine Alkylgruppe, so versteht man darunter geradkettige oder verzweigte Alkylketten mit 1-6 C-Atomen. Besonders bevorzugt ist die Methyl-, Ethyl-, Propyl- und die Butylgruppe.

Bedeutet in der allgemeinen Formel I $R_6$ eine Cycloalkylgruppe, so sind bevorzugt die Cyclopropyl-, Cyclobutyl-, Cyclopentyl- und die Cyclohexylgruppe.

Bilden in der allgemeinen Formel I $R_5$ und $R_6$ zusammen mit dem C-Atom, an das sie gebunden sind, einen Cycloalkylring, so sind bevorzugt der Cyclopropyl-, Cyclobutyl-, Cyclopentyl- und Cyclohexylring.

Für den Fall, daß $R_1$ in ortho-Position des Phenylrings steht, kommen im Sinne der vorliegenden Erfindung insbesondere für $R_1$ ein Wasserstoff- oder Chloratom, eine $C_1$-$C_4$-Alkoxy-, wie z.B. Methoxy-, und die Nitrogruppe in Frage.

Stellt $R_2$ den Substituenten in meta-Position dar, so ist dies insbesondere ein Wasserstoff- oder Chloratom, oder eine $C_1$-$C_4$-Alkoxy- wie z.B. Methoxygruppe.

Für den Fall, daß $R_3$ in para-Position des Phenylrings steht, kommen insbesondere folgende Gruppen in Frage: ein Wasserstoff- oder Halogenatom, eine $C_1$-$C_4$-Alkyl-, z.B. Methyl-; eine $C_2$-$C_4$-Alkenyl-, wie z.B. Allyl-; eine Nitro-; Amino-; Hydroxy-; Cyano-; $C_1$-$C_4$-Alkoxy-, wie z.B. Methoxy-, n-Propyloxy-; Benzyloxy-; 3-Pyridinyloxy-; $C_1$-$C_4$-Alkylsulfonyloxy-, wie z.B. Methylsulfonyloxy-; Hydroxy-$C_1$-$C_4$-alkyloxy-, wie z.B. Hydroxyethyloxy-; Carboxy-$C_1$-$C_4$-alkoxy-, wie z.B. Carboxymethyloxy- oder Carboxypropyloxy-; eine $C_1$-$C_4$-Alkoxycarbonyl-$C_1$-$C_4$-alkoxy-, wie z.B. Ethoxycarbonylmethyloxy- oder Ethoxycarbonylpropyloxy-; eine Di-$C_1$-$C_4$-alkylamino-, wie z.B. Dimethylamino-; oder N-$C_5$-$C_6$-Cycloalkyl-N-$C_1$-$C_4$-alkyl-aminocarbonyl-$C_1$-$C_4$-alkoxy-, wie z.B. N-Cyclohexyl-N-methyl-aminocarbonyl-propyloxygruppe.

Für $R_4$ kommt insbesondere die Methyl- oder Cyanogruppe in Frage. In der Definition von $R_5$ ist die $C_1$-$C_4$-Alkylgruppe, wie z.B. Methylgruppe oder ein Wasserstoffatom bevorzugt.

Für $R_6$ eignet sich vor allem die Alkyl-, wie z.B. Methylgruppe. $R_5$ und $R_6$ können aber auch gemeinsam mit dem C-Atom, an das sie gebunden sind, einen Cyclopentyl- oder Cyclohexylring darstellen.

Die bevorzugte Bedeutung von X ist ein Valenzstrich.

Besonders bevorzugte Arzneimittel enthalten Verbindungen der allgemeinen Formel I,
in der

$R_1$ ein Wasserstoffatom, die Nitro-, Amino-, Fluor-, Chlor-, Brom-, Dimethylamino-, Diethylamino-, Cyano-, Methyl-, Ethyl-, n-Propyl-, i-Propyl-, n-Butyl-, i-Butyl-, tert.-Butyl-, Allyl-, Hydroxy-, Methoxy-, Ethoxy-, Propyloxy-, Allyloxy-, Mercapto-, Methylthio-, Methylsulfinyl-, Methylsulfonyl-, Methylsulfonyloxy-, Cyanmethyloxy-, Benzyloxy-, Pyridinyloxy-, Ethoxycarbonylmethyloxy-, Hydroxymethyl-, Formylamino-, Acetylamino- und die Trifluormethylsulfonylaminogruppe bedeuten kann,

$R_2$ ein Wasserstoffatom, die Methyl-, Ethyl-, n-Propyl-, i-Propyl-, tert.-Butyl-, Chlor-, Methoxy-, Hydroxy- oder Dimethylaminogruppe bedeutet,
$R_3$ ein Wasserstoffatom, die Hydroxy- oder Methoxygruppe bedeutet,
X eine Bindung, die Ethylengruppe oder für den Fall, daß A die Carbonylgruppe bedeutet, auch die Vinylengruppe bedeutet,
A und B verschieden sind und die Imino- und die Carbonylgruppe bedeuten,
$R_4$ die Methyl-, Cyano-, Aminocarbonyl- oder Aminomethylgruppe bedeutet,
$R_5$ ein Wasserstoffatom, die Methyl- oder Ethylgruppe bedeutet,
$R_6$ die Methylgruppe, Ethylgruppe oder Cyclopentylgruppe bedeutet, oder
$R_5$ und $R_6$ zusammen mit dem C-Atom, an das sie gebunden sind, die Cyclopentylgruppe bedeuten.

Gegenstand der vorliegenden Anmeldung sind auch neue Verbindungen der Formel I

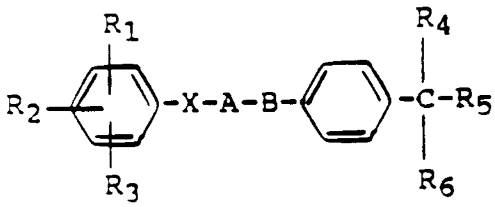

(I)

in der
$R_1$ in ortho-Position des Phenylringes steht und ein Wasserstoffatom oder eine $C_1$-$C_4$-Alkoxy- oder Nitrogruppe bedeutet,
$R_2$ in meta-Position des Phenylringes steht und ein Wasserstoffatom oder eine $C_1$-$C_4$-Alkoxygruppe bedeutet,
$R_3$ in para-Position des Phenylringes steht und eine $C_2$-$C_4$-Alkenyl-, Hydroxy-, Cyano-, $C_1$-$C_4$-Alkoxy-, Benzyloxy-, 3-Pyridinyloxy-, $C_1$-$C_4$-Alkylsulfonyloxy-, Hydroxy-$C_1$-$C_4$-alkoxy-, Carboxy-$C_1$-$C_4$-alkoxy-, $C_1$-$C_4$-Alkoxycarbonyl-$C_1$-$C_4$-alkoxy, Di-$C_1$-$C_4$-Alkylamino- oder N-$C_5$-$C_6$-Cycloalkyl-N-$C_1$-$C_4$-alkyl-aminocarbonyl-$C_1$-$C_4$-alkoxygruppe bedeutet,
X eine Bindung, eine Alkylengruppe, oder für den Fall, daß A die Carbonylgruppe -CO- bedeutet, auch die Vinylengruppe bedeuten kann, und
A und B verschieden sind und die Carbonylgruppe -CO- oder die Iminogruppe -NH- bedeuten, und
$R_4$ eine Methyl-, Cyano-, Aminocarbonyl- oder Aminomethylgruppe bedeutet,
$R_5$ ein Wasserstoffatom, oder eine Methylgruppe bedeutet,
$R_6$ eine Alkyl- oder Cycloalkylgruppe bedeutet,
oder
$R_5$ und $R_6$ zusammen mit dem C-Atom, an das sie gebunden sind, einen Cycloalkylring bedeuten,
deren Tautomere und deren physiologisch verträglichen Salze sowie deren optische Isomeren, mit Ausnahme folgender Verbindungen:
4-(1,1-Dimethylethyl)-N-[4-(1-methylethoxy)phenyl]benzamid

Die erfindungsgemäßen Verbindungen der allgemeinen Formel I können nach an sich bekannten Methoden hergestellt werden. Von besonderem Vorteil ist die Synthese aus Anilinen und Benzoesäuren, wie sie in Schema 1 und Schema 2 dargestellt ist. Für Verbindungen der allgemeinen Formel I, in der A die Aminogruppe -NH- bedeutet und B die Carbonylgruppe -CO- (= allgemeine Formel Ia), setzt man die Aniline der allgemeinen Formel II, in der $R_1$, $R_2$, $R_3$ und X die oben genannten Bedeutungen haben, mit den Benzoesäuren der allgemeinen Formel III um, in der $R_4$, $R_5$ und $R_6$ die oben genannten Bedeutungen haben und Y die Hydroxygruppe, ein Halogenid wie Chlorid oder Bromid, eine Alkyoxy- oder Aryloxygruppe, eine Alkylcarbonyloxy- oder eine Arylcarbonyloxygruppe bedeutet (Schema 1):

Schema 1

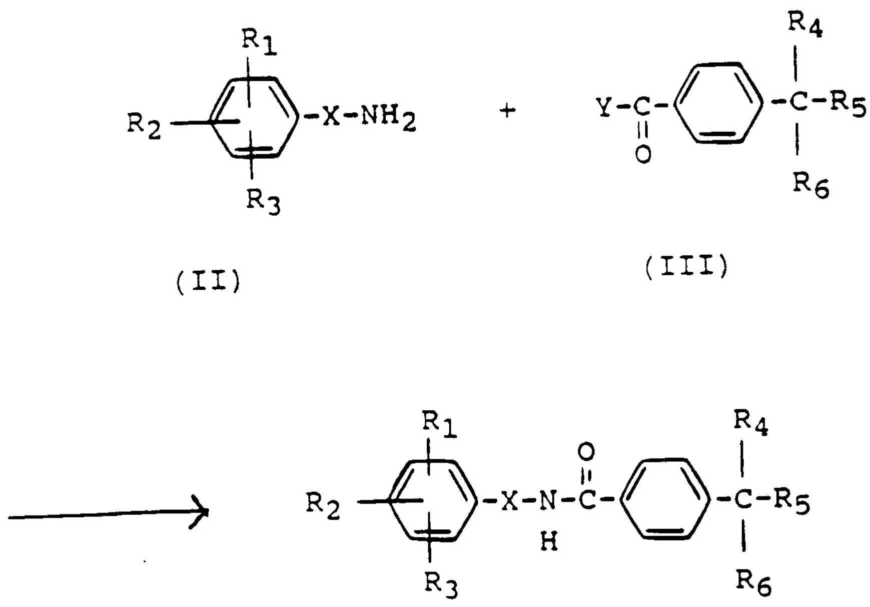

(II) (III)

(I a)

Für Verbindungen der allgemeinen Formel I, in der A die Carbonylgruppe -CO- und B die Aminogruppe -NH- bedeutet (= allgemeine Formel Ib), setzt man die Benzoesäuren der allgemeinen Formel IV, in der $R_1$, $R_2$, $R_3$, X und Y die oben angegebenen Bedeutungen haben, mit den Anilinen der allgemeinen Formel V um, in der $R_4$, $R_5$ und $R_6$ die oben angegebenen Bedeutungen haben (Schema 2).

Schema 2

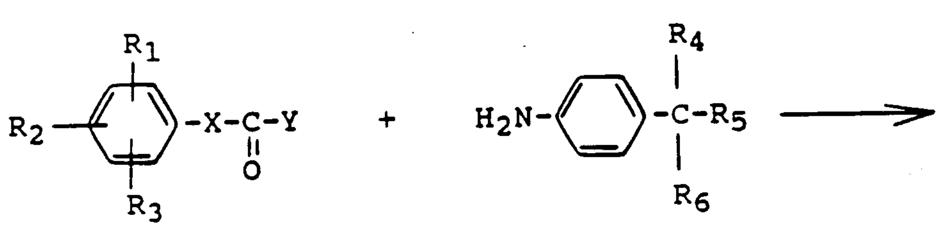

(IV) (V)

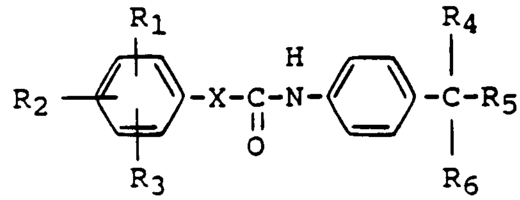

(I b)

Einen Überblick über die Verfahren zur Herstellung von Verbindungen der allgemeinen Formel I benutzten Methoden geben D. Döpp und H. Döpp in J. Falbe (Herausgeber), Methoden der Organischen Chemie (Houben-Weyl), Verlag Thieme, Stuttgart, New York, 1985, S. 934 ff.

Bedeutet in der allgemeinen Formel III oder IV Y die Hydroxygruppe, so sind die Verbindung der allgemeinen Formel III oder IV Carbonsäuren. Die bevorzugte Methode zur Umsetzung mit den Aminen, der allgemeinen Formel II oder V besteht in der Reaktion von etwa äquimolaren Mengen des Amins und der Säure in Gegenwart eines wasserentziehenden Mittels. Dafür kommt beispielsweise Polyphosphorsäure in Betracht, die dann gleichzeitig als Lösungsmittel dient. Die Reaktionen laufen zwischen 50 °C und 200 °C ab. Die Endprodukte der allgemeinen Formel I fallen im allgemeinen nach Zugabe von Wasser aus und werden nach Filtration durch Umkristallisation oder säulenchromatographisch gereinigt. Eine weitere bevorzugte Methode zur Herstellung von Verbindungen der allgemeinen Formel I besteht in der Umsetzung von etwa äquimolaren Mengen des Amins und der Säure in einem geeigneten Lösungsmittel mit etwa einer äquivalenten Menge eines Halogenierungsmittels, wie Phosphortrichlorid, Phosphorpentachlorid oder Thionylchlorid, bei Temperaturen zwischen Raumtemperatur und der Rückflußtemperatur der Mischung. Geeignete Lösungsmittel sind Methylenchlorid Tetrachlorkohlenstoff, Diethylether, Toluol, Xylol oder Chlorbenzol. Im allgemeinen fällt das Produkt aus der Lösung aus und wird durch Filtration gewonnen. Falls erforderlich, kann die Reaktionsmischung konzentriert werden, bis zu einem Punkt, bei dem das Produkt aus der Lösung auszufallen beginnt. Als weitere Kondensationsmittel bei dieser Reaktion kommen saure Kationenaustauscher, Sulfoniumsalze, Schwefelsäurehalogenide, 2-Halogenpyridiniumsalze, Phosphoniumsalze und Dicyclohexylcarbodiimid in Betracht.

Bedeutet in der allgemeinen Formel III oder IV Y eine Alkoxy-oder Aryloxygruppe, so handelt es sich bei den Verbindungen der allgemeinen Formel III oder IV um Carbonsäureester. Man arbeitet in Gegenwart oder Abwesenheit spezieller Lösungsmittel bei Temperaturen im Bereich von 20 °C bis zur Siedehitze des Gemisches. Bevorzugt ist dabei die Reaktion etwa äquimolarer Mengen des Amins und des Esters in Polyphosphorsäure bei 50 °C - 200 °C, jedoch kann man auch in einem inerten Lösungsmittel wie Methylenchlorid, Benzol, Toluol, Chlorbenzol am besten in Gegenwart von etwas mehr als einem Äquivalent einer Base wie Natriumethanolat oder Butyllithium, oder von Natriumhydrid in Dimethylsulfoxid arbeiten.

Bedeutet in der allgemeinen Formel III oder IV Y eine Alkylcarbonyloxy- oder Arylcarbonyloxygruppe, so handelt es sich bei den Verbindungen der allgemeinen Formel III oder IV um Anhydride. Da Anhydride im allgemeinen reaktiver sind als Carbonsäuren oder Carbonsäureester, kann man die Umsetzung mit den Aminen der allgemeinen Formel II oder V schon bei etwas niedrigeren Temperaturen vornehmen. Bevorzugt arbeitet man in einem inerten Lösungsmittel wie Dichlormethan, Diethylether, Benzol, Toluol, bei Temperaturen zwischen Raumtemperatur und 60 °C. Man gibt dabei das Amin und das Anhydrid in etwa äquimolaren Mengen zusammen, wobei im allgemeinen eine exotherme Reaktion einsetzt. Nach Abklingen wird zur Vervollständigung der Reaktion noch einige Zeit gelinde erwärmt.

Bedeutet in der allgemeinen Formel III oder IV Y ein Halogenid, so handelt es sich bei den Verbindungen der allgemeinen Formel III oder IV um Carbonsäurehalogenide. Darunter versteht man in erster Linie Säurechloride und -bromide. Da die Säurehalogenide reaktiver sind als die Carbonsäuren, -ester, oder -anhydride, ist es meist notwendig, die Reaktionsmischung zu kühlen. Man arbeitet am besten bei Temperaturen zwischen -10 °C und Raumtemperatur.

Bevorzugt geht man dabei so vor, daß nach Schotten-Baumann zur wässrigen Lösung des Amins der allgemeinen Formel II oder V, welche noch eine Base wie Alkalihydroxide, Natriumcarbonat oder Pyridin enthält, das Säurechlorid unter Eiskühlung langsam zutropft, und anschließend noch einige Zeit bei Raumtemperatur stehen läßt. Diese Reaktion ist nicht nur in Wasser möglich, sondern auch in organischen Lösungsmitteln wie Methylenchlorid, Ether, Benzol oder Toluol. Auch ohne säurebindende Mittel lassen sich die Amine durch Carbonsäurechloride nahezu quantitativ acylieren, in dem man das Amin und das

Carbonsäurechlorid in einem interten Lösungsmittel wie Methylenchlorid, Benzol oder Toluol bis zur Beendigung der Gasentwicklung kocht, was 1-24 Stunden dauert. Gibt man jedoch ein säurebindendes Mittel wie Triethylamin oder Pyridin in geringem Überschuß zu, so läuft die Reaktion schon bei Temperaturen zwischen -10 ° C und Raumtemperatur ab.

Verbindungen der allgemeinen Formel I können auch in andere Verbindungen der allgemeinen Formel I umgewandelt werden. Dies trifft zu für

a) für die Alkylierung von Verbindungen der allgemeinen Formel I, in der einer oder mehrere der Substituenten $R_1$, $R_2$, $R_3$ eine Hydroxy- oder Mercaptogruppe bedeutet, zu den entsprechenden Alkoxy- oder Alkylthioverbindungen. Diese Reaktionen werden vorzugsweise in einem Lösungsmittel wie Aceton, Ether, Benzol, Toluol oder Dimethylformamid bei Temperaturen zwischen -30 ° C und + 100 ° C, vorzugsweise bei Raumtemperatur in Gegenwart einer Base wie Kaliumhydroxid und eines Alkylierungsmittels wie Alkylhalogeniden oder Alkylsulfaten durchgeführt,

b) für die Herstellung von Verbindungen der allgemeinen Formel I, in der $R_1$ eine Alkylsulfinyl- oder Alkysulfonylgruppe darstellt, durch nachträgliche Oxidation einer Verbindung in der $R_1$ eine Alkylmercaptogruppe ist. Die Oxidation wird vorzugsweise in einem Lösungsmittel oder Lösungsmittelgemisch, z.B. Wasser, Wasser/Pyridin, Aceton, Eisessig, verdünnter Schwefelsäure oder Trifluoressigsäure, je nach dem verwendeten Oxidationsmittel zweckmäßigerweise bei Temperaturen zwischen -80 ° C und 100 ° C durchgeführt.

Zur Herstellung einer Alkylsulfinylverbindung der allgemeinen Formel I wird die Oxidation zweckmäßigerweise mit einem Aequivalent des verwendeten Oxidationsmittels durchgeführt, z.B. mit Wasserstoffperoxid in Eisessig, Trifluoressigsäure oder Ameisensäure bei 0 bis 20 ° C oder in Aceton bei 0 bis 60 ° C, mit einer Persäure wie Perameisensäure in Eisessig oder Trifluoressigsäure bei 0 bis 50 ° C oder mit m-Chlorperbenzoesäure in Methylenchlorid oder Chloroform bei -20 bis 60 ° C, mit Natriummetaperjodat in wässrigem Methanol oder Ethanol bei -15 bis 25 ° C, mit Brom in Eisessig oder wässriger Essigsäure, mit N-Bromidsuccinimid in Ethanol, mit tert.-Butyl-hypochlorit in Methanol bei -80 bis -30 ° C, mit Jodbenzodichlorid in wässrigem Pyridin bei 0 bis 50 ° C, mit Salpetersäure in Eisessig bei 0 bis 20 ° C, mit Chromsäure in Eisessig oder in Aceton bei 0 bis 20 ° C und mit Sulfurylchlorid in Methylenchlorid bei -70 ° C, der hierbei erhaltene Thioether-Chlorkomplex wird zweckmäßigerweise mit wässrigem Ethanol hydrolysiert.

Zur Herstellung einer Alkylsulfonylverbindung der allgemeinen Formel I wird die Oxidation zweckmäßigerweise mit einem bzw. mit zwei oder mehr Aequivalenten des verwendeten Oxidationsmittels durchgeführt, z.B. Wasserstoffperoxid in Eisessig, Trifluoressigsäure oder in Ameisensäure bei 20 bis 100 ° C oder in Aceton bei 0 bis 60 ° C,. mit einer Persäure wie Perameisensäure oder m-Chlorperbenzoesäure in Eisessig, Trifluoressigsäure, Methylenchlorid oder Chloroform bei Temperaturen zwischen 0 und 60 ° C, mit Salpetersäure in Eisessig bei 0 bis 20 ° C, mit Chromsäure oder Kaliumpermanganat in Eisessig, Wasser/Schwefelsäure oder in Aceton bei 0 bis 20 ° C.

c) für die Herstellung von Verbindungen der allgemeinen Formel I, in der $R_1$ eine Alkansulfonyloxy-, Trifluormethansulfonyloxy-,Alkansulfonylamino- oder Trifluormethansulfonylaminogruppe darstellt, durch die nachträgliche Umsetzung einer Verbindung in der $R_1$ eine Hydroxygruppe mit einer Sulfonsäure der allgemeinen Formel VI

$R_7$-$SO_3H$ (VI) ,

in der $R_7$ eine Alkyl- oder eine Trifluormethylgruppe darstellt, in Gegenwart eines wasserentziehenden und/oder die Säure oder das Amin aktivierenden Mittels oder mit deren reaktionsfähigen Derivaten.

Die Umsetzung wird zweckmäßigerweise in einem Lösungsmittel oder Lösungsmittelgemisch wie Methylenchlorid, Ether, Tetrahydrofuran, Dioxan oder Benzol gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, in Gegenwart eines die Säure aktivierenden oder wasserentziehenden Mittels wie Thionylchlorid oder Phosphorpentachlorid, vorzugsweise jedoch mit einem reaktionsfähigen Derivat einer Verbindung der allgemeinen Formel VII z.B. mit deren Anhydrid oder Halogenid wie Methansulfonsäurechlorid oder Ethansulfonsäurechlorid, vorzugsweise bei Temperaturen zwischen 0 und 100 ° C, z.B. bei Temperaturen zwischen Raumtemperatur und 50 ° C, durchgeführt.

d) für die Herstellung von Verbindungen der allgemeinen Formel I, in der $R_1$ eine durch eine Amino-, Alkylamino-oder Dialkylaminogruppe substituierte Formylgruppe darstellt, durch die nachträgliche Umsetzung einer Verbindung in der $R_1$ eine Carboxylgruppe darstellt, oder einem reaktionsfähigen Derivat hiervon wie z.B. Ester oder Säurechlorid mit einem Amin der allgemeinen Formel VII

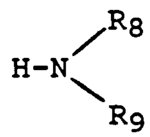

(VII)

in der $R_8$ und $R_9$, die gleich oder verschieden sein können, Wasserstoffatome oder Alkylgruppen darstellen, oder mit einem reaktionsfähigen Derivat hiervon, falls $R_1$ die Carboxylgruppe darstellt. Die Umsetzung wird zweckmäßigerweise in einem Lösungsmittel wie Methylenchlorid, Ethanol, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureethylester, Thionylchlorid, Phosphortrichlorid, Phosphorpentoxid, N,N′-Dicyclohexylcarbodiimid/N-Hydroxysuccinimid, N,N′-Carbonyldiimidazol oder N,N′-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiaeren organischen Base wie Triethylamin oder Pyridin, welche gleichzeitig als Lösungsmittel dienen können, bei Temperaturen zwischen -25 und 250 °C, vorzugsweise jedoch bei Temperaturen zwischen -10 °C und der Siedetemperatur des verwendeten Lösungsmittels durchgeführt, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Besonders vorteilhaft wird jedoch die Umsetzung mit einem entsprechenden Halogenid, z.B. dem Carbonsäure-chlorid und einem entsprechenden Amin, wobei dieses gleichzeitig als Lösungsmittel dienen kann, und bei Temperaturen zwischen 0 und 50 °C durchgeführt.

e) die Verseifung von Verbindungen der allgemeinen Formel I, in der $R_1$ oder $R_4$ eine Cyanogruppe bedeutet, zu Verbindungen der allgemeinen Formel I, in der $R_1$ oder $R_4$ die Aminocarbonylgruppe bedeuten. Man arbeitet in Gegenwart einer Säure wie Salzsäure, Schwefelsäure, Phosphorsäure oder Trichloressigsäure, oder in Gegenwart einer Base wie Natriumhydroxid oder Kaliumhydroxid in einem geeigneten Lösungsmittel wie Wasser, Methanol, Wasser/Methanol, Wasser/Ethanol, Ethanol, Wasser/Isopropanol oder Wasser/Dioxan bei Temperaturen zwischen Raumtemperatur und der Siedetemperatur des Reaktionsgemisches, oder in einem inerten Lösungsmittel wie Dichlormethan unter Zusatz eines Phasentransfer-Katalysators wie Tetrabutylammoniumhydrogensulfat, unter Zusatz von Wasserstoffperoxid, wobei man bei Raumtemperatur arbeiten kann,

f) die Reduktion von Verbindungen der allgemeinen Formel I, in der einer der Substituenten $R_1$, $R_2$ oder $R_3$ eine Nitrogruppe bedeutet, zu Verbindungen der allgemeinen Formel I, in der $R_1$, $R_2$ oder $R_3$ eine Aminogruppe bedeuten. Die Hydrierung wird vorzugsweise in einem Lösungsmittel wie Wasser, Ethanol, Eiessig, Essigester oder Dimethylformamid mit Wasserstoff in Gegenwart eines Katalysators wie Raney-Nickel, Platin oder Palladium/Kohle, mit Metallen wie Eisen, Zinn, oder Zink in Gegenwart einer Säure, mit Salzen wie Eisen(II)sulfat, Zinn(II)chlorid, Natriumsulfid, Natriumhydrogensulfid oder Natriumdithionit, oder mit Hydrazin in Gegenwart von Raney-Nickel bei Temperaturen zwischen 0 und 250 °C, vorzugsweise jedoch bei Raumtemperatur, durchgeführt.

g) die Hydrierung einer Verbindung der allgemeinen Formel I, in der X die Vinylengruppe bedeutet, zu Verbindungen der allgemeinen Formel I, in der X die Ethylengruppe bedeutet.

Die Hydrierung wird vorzugsweise in einem Lösungsmittel wie Wasser, Wasser/Ethanol, Methanol, Eisessig, Essigester, Dimethylformamid, in Gegenwart von Wasserstoff und einem Katalysator wie Raney-Nickel, Platin oder Palladium/Kohle durchgeführt.

h) die Reduktion von Verbindungen der allgemeinen Formel I, in der $R_4$ eine Cyanogruppe bedeutet, zu Verbindungen der allgemeinen Formel I, in der $R_4$ eine Aminomethylgruppe bedeutet. Diese Reduktion führt man mit Wasserstoff unter Druck in Gegenwart von Ammoniak und einem Katalysator wie Raney-Nickel durch, vorzugsweise bei Temperaturen zwischen 50 °C und 150 °C und Drücken von 50-250 bar.

i) die Reduktion einer Verbindung der allgemeinen Formel I, in der einer der Substituenten $R_1$, $R_2$ oder $R_3$ eine Alkyloxycarbonylgruppe enthält zur entsprechenden Hydroxyalkylverbindung. Diese Reduktion führt man vorzugsweise mit Lithiumaluminiumhydrid in einem Ether wie Diethylether oder Dioxan am Siedepunkt des Lösungsmittels durch.

j) die Verseifung einer Verbindung der allgemeinen Formel I, in der einer der Substituenten $R_1$, $R_2$ oder $R_3$ eine Alkoxycarbonylgruppe enthält zur entsprechenden Hydroxycarbonylverbindung. Solche Verseifungen führt man vorzugsweise in wässriger oder wässrig-alkoholischer Natronlauge oder Kaliumhydroxidlösung durch, und setzt dann die Säure durch Ansäuern mit einer Mineralsäure frei.

k) die Nitrierung von Verbindungen der allgemeinen Formel I, in der X eine Bindung darstellt, A eine Aminogruppe -NH-und B die Carbonylgruppe -CO-. Dabei entstehen Verbindungen, in der einer der Substituenten $R_1$, $R_2$ oder $R_3$ die Nitrogruppe darstellt, die in ortho-Stellung zur Amidfunktion A-B (= -NH-CO-) eintritt.

Die Nitrierung führt man bevorzugt mit Salpetersäure in Schwefelsäure bei Temperaturen zwischen -20 °C und +50 °C durch. Sie kann jedoch auch ohne Schwefelsäure, oder an deren Stelle in Wasser, Eisessig oder Acetanhydrid durchgeführt werden, oder mit $N_2O_5$ in $CCl_4$ in Gegenwart von $P_2O_5$. Als Nitrierungsreagenzien können auch Anhydride wie Acetylnitrat, oder Nitrylhalogenide mit $FeCl_3$, Methylnitrat und $BF_3$, oder Nitroniumsalze wie $NO_2BF_4$, $NO_2PF_6$ oder $NO_2CF_3SO_3$ dienen. Zur Nitrierung kann auch eine Mischung aus Salpetersäure und salpetriger Säure benutzt werden, welche $N_2O_4$ als eigentliche nitrierende Spezies liefert.

Die als Vorstufen zur Herstellung von Verbindungen der allgemeinen Formel I gemäß Schema 1 und Schema 2 benötigten Aniline der allgemeinen Formel II und der Benzoesäure der allgemeinen Formel IV sind käuflich oder literaturbekannt. Von den Anilinen der allgemeinen Formel V ist 4-tert.Butylanilin käuflich, die anderen sind literaturbekannt (vgl. Jönsson, Acta chem. scand. 8 (1954) 1211; Nerdel, Würgau, Liebigs Ann. Chem. 621 (1959) 34), oder können nach den darin beschriebenen Methoden hergestellt werden.

Schema III

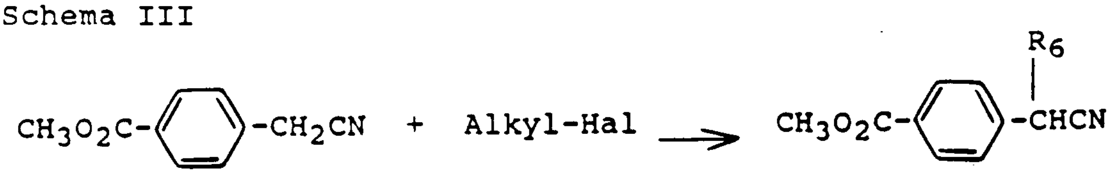


(VIII) (IX) (III a)

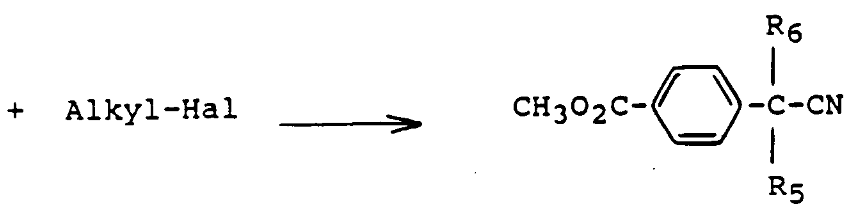


(X) (III b)

Verbindungen der allgemeinen Formel III, in der $R_4$ die Cyanogruppe bedeutet, $R_5$ und $R_6$ die oben angegebenen Bedeutungen haben und Y die Alkoxygruppe bedeutet (= allgemeine Formeln III a und III b) stellt man durch schrittweise Alkylierung von 4-(Cyanomethyl)benzoesäureestern her, z.B. der Verbindung VIII, mit den Alkylierungsmitteln der allgemeinen Formeln IX und X, in denen Alkyl eine $C_1$-$C_6$-Alkylgruppe oder eine $C_3$-$C_6$-Cycloalkylgruppe bedeutet und Hal ein Halogenatom, vorzugsweise Chlorid oder Bromid bedeutet. Die Verbindungen der allgemeinen Formeln IX oder X können gleich oder verschieden sein. Bilden in der allgemeinen Formel III $R_5$ und $R_6$ zusammen mit dem C-Atom, an das sie gebunden sind, einen Cycloalkylring und $R_4$ die Cyanogruppe und Y die Alkoxygruppe (= allgemeinen Formel III c), so führt man die Alkylierung der Verbindung VIII mit dem Alkylendihalogenid der allgemeinen Formel XI durch:

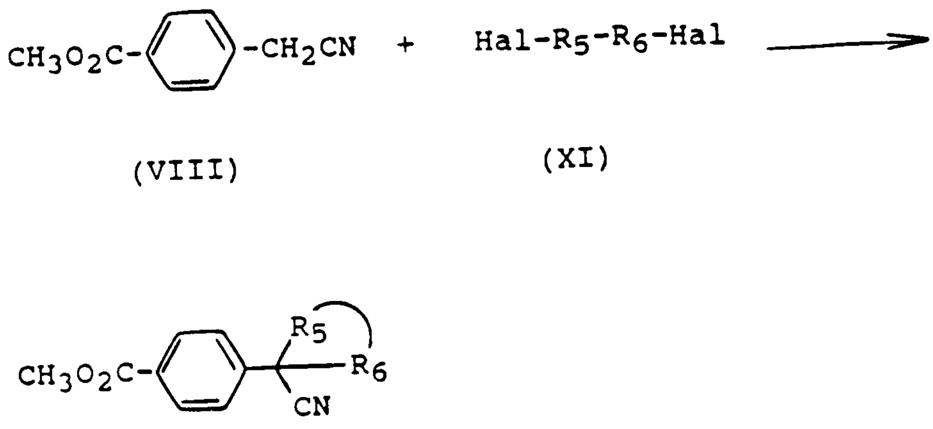

(VIII) (XI)

(III c)

Diese Alkylierungen führt man in einem inerten Lösungsmittel wie Dichlormethan, Ether, Toluol, Xylol in Gegenwart einer Base wie Natronlauge oder Kalilauge, gewünschtenfalls in Gegenwart eines Phasentransfer-Katalysators wie Tetrabutylammoniumbromid und einem Äuivalent des Alkylierungsmittels IX, X oder XI, durch.

Die Verbindung VIII ist literaturbekannt: J.F.Codington, E. Mosettig, J.Org.Chem. 17 (1952), 1035.

Ein weiteres Verfahren zur Herstellung von Verbindungen der allgemeinen Formel III geht aus von den Anilinen der allgemeinen Formel V

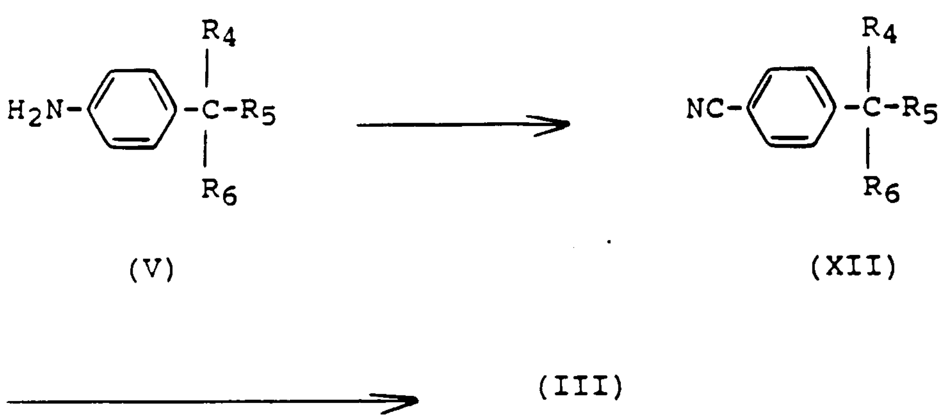

(V) (XII)

(III)

Durch die Reaktionsfolge Diazotierung, Cyanierung (Sandmeyer-Reaktion) und Verseifung des Nitrils können die Verbindungen der allgemeinen Formel III gewonnen werden.

Die Verbindungen der Formel I können auch Ausgangsverbindungen zur Herstellung von anderen pharmazeutisch wirksamen Verbindungen sein, die Gegenstand der deutschen Patentanmeldung P 3830060.5 sind.

Zur Herstellung von Arzneimitteln werden die Substanzen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungs-mittel, Lösungsvermittler oder Puffer enthält.

Derartige Zusätze sind z.B. Tartrat- und Citratpuffer, Ethanol, Komplexbildner (wie Ethylendiamintetraessigsäure und deren nicht toxische Salze) und hochmolekulare Polymere (wie flüssiges Polyethylenoxid) zur

Viskositätsregulierung. Feste Trägerstoffe sind z.B. Stärke, Lactose, Mannit, Methylcellulose, Talkum, hochdisperse Kieselsäuren, hochmolekulare Fettsäuren (wie Staerinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die Verbindungen der Formel I können gewünschtenfalls in ihre physiologisch verträgliche Säureadditionssalze mit anorganischen oder organischen Säuren überführt werden. Als Säuren kommen beispielsweise Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure in Betracht.

Die Verbindungen werden üblicherweise in Mengen von 10-1500 mg pro Tag bezogen auf 75 kg Körpergewicht appliziert. Bevorzugt ist es, 2-3 mal pro Tag 1-2 Tabletten mit einem Wirkstoffgehalt von 5-500 mg zu verabreichen. Die Tabletten können auch retardiert sein, wodurch nur noch 1 mal pro Tag 1-2 Tabletten mit 20-700 mg Wirkstoff gegeben werden müssen. Der Wirkstoff kann auch durch Injektion 1-8 mal pro Tag bzw. durch Dauerinfusion gegeben werden, wobei Mengen von 10-1000 mg pro Tag normalerweise ausreichen.

Bevorzugt im Sinne der Erfindung sind außer den in den Beispielen genannten Verbindungen die folgenden:

N-(2-Hydroxyphenyl)-4-(1,1-dimethylethyl)benzamid
N-(2-(Methoxycarbonyl)phenyl)-4-(1,1-dimethylethyl)benzamid
N-(2-Ethylphenyl)-4-(1,1-dimethylethyl)benzamid
N-(2-Mercaptophenyl)-4-(1,1-dimethylethyl)benzamid
N-(2-Cyanophenyl)-4-(1,1-dimethylethyl)benzamid
N-(2-(Ethoxy)phenyl)-4-(1,1-dimethylethyl)benzamid
N-(2-Methylphenyl)-4-(1,1-dimethylethyl)benzamid
N-(2-(sec.Butyl)phenyl)-4-(1,1-dimethylethyl)benzamid
N-(2-(Hydroxymethyl)phenyl)-4-(1,1-dimethylethyl)benzamid
N-(2-Carboxyphenyl)-4-(1,1-dimethylethyl)benzamid
N-(2-Ethoxycarbonyl)phenyl)-4-(1,1-dimethylethyl)benzamid
N-(2-Biphenyl)-4-(1,1-dimethylethyl)benzamid
N-(2-Fluorphenyl)-4-(1,1-dimethylethyl)benzamid
N-(2-Nitrophenyl)-4-(1,1-dimethylethyl)benzamid
N-(2-Aminophenyl)-4-(1,1-dimethylethyl)benzamid
N-(2-Bromphenyl)-4-(1,1-dimethylethyl)benzamid
N-(2-Iodphenyl)-4-(1,1-dimethylethyl)benzamid
N-(2-Acetylphenyl)-4-(1,1-dimethylethyl)benzamid
N-(2-Allyloxyphenyl)-4-(1,1-dimethylethyl)benzamid
N-(3-Carboxylphenyl)-4-(1,1-dimethylethyl)benzamid
N-(3-Hydroxyphenyl)-4-(1,1-dimethylethyl)benzamid
N-(3-Nitrophenyl)-4-(1,1-dimethylethyl)benzamid
N-(3-Aminophenyl)-4-(1,1-dimethylethyl)benzamid
N-(3-Methylphenyl)-4-(1,1-dimethylethyl)benzamid
N-(3-Ethylphenyl)-4-(1,1-dimethylethyl)benzamid
N-(3-Cyanophenyl)-4-(1,1-dimethylethyl)benzamid
N-(3-Hydroxymethylphenyl)-4-(1,1-dimethylethyl)benzamid
N-(3-Acetylphenyl)-4-(1,1-dimethylethyl)benzamid
N-(3-Dimethylaminophenyl)-4-(1,1-dimethylethyl)benzamid
N-(3-Fluorphenyl)-4-(1,1-dimethylethyl)benzamid
N-(3-Iodphenyl)-4-(1,1-dimethylethyl)benzamid
N-(3-Ethyloxyphenyl)-4-(1,1-dimethylethyl)benzamid
N-(3-Bromphenyl)-4-(1,1-dimethylethyl)benzamid
N-(3-Allyloxyphenyl)-4-(1,1-dimethylethyl)benzamid
N-(4-Acetylaminophenyl)-4-(1,1-dimethylethyl)benzamid
N-(4-Acetylphenyl)-4-(1,1-dimethylethyl)benzamid
N-(4-Hydroxyphenyl)-4-(1,1-dimethylethyl)benzamid
N-(4-Carboxymethylphenyl)-4-(1,1-dimethylethyl)benzamid
N-(4-Ethylphenyl)-4-(1,1-dimethylethyl)benzamid
N-(4-Aminocarbonylphenyl)-4-(1,1-dimethylethyl)benzamid
N-(4-Bromphenyl)-4-(1,1-dimethylethyl)benzamid

N-(4-Ethyloxyphenyl)-4-(1,1-dimethylethyl)benzamid
N-(4-sec.Butylphenyl)-4-(1,1-dimethylethyl)benzamid
N-(4-Butylphenyl)-4-(1,1-dimethylethyl)benzamid
N-(4-Isopropylphenyl)-4-(1,1-dimethylethyl)benzamid
N-<4-[N′-Ethyl-N′-(2-hydroxyethyl)aminophenyl]>-4-(1,1-dimethylethyl)benzamid
N-(4-Propinylphenyl)-4-(1,1-dimethylethyl)benzamid
N-(4-Diethylaminophenyl-4-(1,1-dimethylethyl)benzamid
N-(4-Fluorphenyl)-4-(1,1-dimethylethyl)benzamid
N-(4-Iodphenyl)-4-(1,1-dimethylethyl)benzamid
N-(4-Methylphenyl)-4-(1,1-dimethylethyl)benzamid
N-(4-Sulfonylphenyl)-4-(1,1-dimethylethyl)benzamid
N-(4-Carboxyphenyl)-4-(1,1-dimethylethyl)benzamid
N-(4-Allyloxyphenyl)-4-(1,1-dimethylethyl)benzamid
N-(3-Chlor-2-methylphenyl)-4-(1,1-dimethylethyl)benzamid
N-(2-Methyl-3-nitrophenyl)-4-(1,1-dimethylethyl)benzamid
N-(2-Methyl-3-aminophenyl)-4-(1,1-dimethylethyl)benzamid
N-(5,6,7,8-Tetrahydronaphthalin-1-yl)-4-(1,1-dimethylethyl)benzamid
N-(2,3-Dichlorphenyl)-4-(1,1-dimethylethyl)benzamid
N-(2,3-Dimethylphenyl)-4-(1,1-dimethylethyl)benzamid
N-(4-Hydroxy-2-methylphenyl)-4-(1,1-dimethylethyl)benzamid
N-(2,4-Dimethoxyphenyl)-4-(1,1-dimethylethyl)benzamid
N-(2-Chlor-4-nitrophenyl)-4-(1,1-dimethylethyl)benzamid
N-(2-Chlor-4-aminophenyl)-4-(1,1-dimethylethyl)benzamid
N-(4-Chlor-2-nitrophenyl)-4-(1,1-dimethylethyl)benzamid
N-(4-Chlor-2-aminophenyl)-4-(1,1-dimethylethyl)benzamid
N-(4-Chlor-2-methylphenyl)-4-(1,1-dimethylethyl)benzamid
N-(2,4-Dinitrophenyl)-4-(1,1-dimethylethyl)benzamid
N-(2,4-Diaminophenyl)-4-(1,1-dimethylethyl)benzamid
N-(2-Nitro-4-methoxyphenyl)-4-(1,1-dimethylethyl)benzamid
N-(2-Amino-4-methoxyphenyl)-4-(1,1-dimethylethyl)benzamid
N-(4-Nitro-2-methoxyphenyl)-4-(1,1-dimethylethyl)benzamid
N-(4-Amino-2-methoxyphenyl)-4-(1,1-dimethylethyl)benzamid
N-(4-Nitro-2-methylphenyl)-4-(1,1-dimethylethyl)benzamid
N-(4-Amino-2-methylphenyl)-4-(1,1-dimethylethyl)benzamid
N-(2-Hydroxy-4-methylphenyl)-4-(1,1-dimethylethyl)benzamid
N-(2-Brom-4-nitrophenyl)-4-(1,1-dimethylethyl)benzamid
N-(2-Brom-4-aminophenyl)-4-(1,1-dimethylethyl)benzamid
N-(2,4-Dichlorphenyl)-4-(1,1-dimethylethyl)benzamid
N-(2,4-Dimethylphenyl)-4-(1,1-dimethylethyl)benzamid
N-(2,4-Difluorphenhyl-4-(1,1-dimethylethyl)benzamid
N-(4-Methyl-2-chlorphenyl)-4-(1,1-dimethylethyl)benzamid
N-(2,5-Dimethoxyphenyl)-4-(1,1-dimethylethyl)benzamid
N-(2-Hydroxy-5-chlorphenyl)-4-(1,1-dimethylethyl)benzamid
N-(2-Methyl-5-chlorphenyl)-4-(1,1-dimethylethyl)benzamid
N-(2-Methoxy-5-nitrophenyl)-4-(1,1-dimethylethyl)benzamid
N-(2-Methoxy-5-aminophenyl)-4-(1,1-dimethylethyl)benzamid
N-(2-Methyl-5-nitrophenyl)-4-(1,1-dimethylethyl)benzamid
N-(2-Methyl-5-aminophenyl)-4-(1,1-dimethylethyl)benzamid
N-(2,5-Dimethylphenyl)-4-(1,1-dimethylethyl)benzamid
N-(2-Nitro-5-chlorphenyl)-4-(1,1-dimethylethyl)benzamid
N-(2-Amino-5-chlorphenyl)-4-(1,1-dimethylethyl)benzamid
N-(2-Methyl-5-fluorphenyl)-4-(1,1-dimethylethyl)benzamid
N-(2,5-Bis(methoxycarbonyl)phenyl)-4-(1,1-dimethylethyl)benzamid
N-(2,5-Dichlorphenyl)-4-(1,1-dimethylethyl)benzamid
N-(2-Hydroxy-5-methylphenyl)-4-(1,1-dimethylethyl)benzamid
N-(2,6-Dimethylphenyl)-4-(1,1-dimethylethyl)benzamid
N-(2,6-Dichlorphenyl)-4-(1,1-dimethylethyl)benzamid
N-(2-Methyl-6-nitrophenyl)-4-(1,1-dimethylethyl)benzamid

N-(2-Methyl-6-aminophenyl)-4-(1,1-dimethylethyl)benzamid
N-(3-Chlor-4-methylphenyl)-4-(1,1-dimethylethyl)benzamid
N-(3-Methoxy-4-methylphenyl)-4-(1,1-dimethylethyl)benzamid
N-(3,4-Dimethylphenyl)-4-(1,1-dimethylethyl)benzamid
N-(3-Nitro-4-fluorphenyl)-4-(1,1-dimethylethyl)benzamid
N-(3-Amino-4-fluorphenyl)-4-(1,1-dimethylethyl)benzamid
N-(1,4-Benzodioxan-6-yl)-4-(1,1-dimethylethyl)benzamid
N-(3-Chlor-4-fluorphenyl)-4-(1,1-dimethylethyl)benzamid
N-(3,4-Dichlorphenyl)-4-(1,1-dimethylethyl)benzamid
N-(3,5-Dimethylphenyl)-4-(1,1-dimethylethyl)benzamid
N-(3,5-Dimethoxyphenyl)-4-(1,1-dimethylethyl)benzamid
N-(2,4,5-Trichlorphenyl)-4-(1,1-dimethylethyl)benzamid
N-(2,4,6-Trimethylphenyl)-4-(1,1-dimethylethyl)benzamid
N-(2,6-Dichlor-4-nitrophenyl)-4-(1,1-dimethylethyl)benzamid
N-(2,6-Dichlor-4-aminophenyl)-4-(1,1-dimethylethyl)benzamid
N-(2,4,6-Trichlorphenyl)-4-(1,1-dimethylethyl)benzamid
N-(2,4,6-Tribromphenyl)-4-(1,1-dimethylethyl)benzamid
N-(3,5-Dimethyl-4-hydroxyphenyl)-4-(1,1-dimethylethyl)benzamid
N-(4-Allyloxy-2-methylphenyl)-4-(1,1-dimethylethyl)benzamid
N-(2-Allyloxy-4-methylphenyl)-4-(1,1-dimethylethyl)benzamid
N-(2-Allyloxy-5-chlorphenyl)-4-(1,1-dimethylethyl)benzamid
N-(2-Allyloxy-5-methylphenyl)-4-(1,1-dimethylethyl)benzamid
N-(4-Allyloxy-3,5-dimethylphenyl)-4-(1,1-dimethylethyl)benzamid
N-(4-(1,1-Dimethylethyl)phenyl)-(4-methoxyphenyl)benzamid
N-(4-(1,1-Dimethylethyl)phenyl)-4-hydroxy-benzamid
N-(4-(1,1-Dimethylethyl)phenyl)-4-propyloxy-benzamid
N-(4-(1,1-Dimethylethyl)phenyl)-4-allyloxy-benzamid
N-(4-(1,1-Dimethylethyl)phenyl)-4-benzyloxy-benzamid
N-(4-(1,1-Dimethylethyl)phenyl)-4-methylsulfonyloxybenzamid
N-(4-(1,1-Dimethylethyl)phenyl)-2-methoy-benzamid
N-(4-(1,1-Dimethylethyl)phenyl)-3-methoxy-benzamid
N-(4-(1,1-Dimethylethyl)phenyl)-2-chlor-benzamid
N-(4-(1,1-Dimethylethyl)phenyl)-3-chlor-benzamid
N-(4-(1,1-Dimethylethyl)phenyl)-4-chlor-benzamid
N-(4-(1,1-Dimethylethyl)phenyl)-4-methylmercapto-benzamid
N-(4-(1,1-Dimethylethyl)phenyl)-4-methyl-benzamid
N-(4-(1,1-Dimethylethyl)phenyl)-4-cyano-benzamid
N-(4-(1,1-Dimethylethyl)phenyl)-4-nitro-benzamid
N-(4-(1,1-Dimethylethyl)phenyl)-4-dimethylamino-benzamid
N-(4-(1,1-Dimethylethyl)phenyl)-3,4-dimethoxy-benzamid
N-(4-(1,1-Dimethylethyl)phenyl)-3,4-methylendioxy-benzamid
N-(4-(1,1-Dimethylethyl)phenyl)-4-(3-pyridinyoxy-benzamid
N-(4-(1,1-Dimethylethyl)phenyl)-4-amino-benzamid
N-(4-(1-Cyano-cyclopentyl)phenyl)-4-methoxy-benzamid
N-(4-(1-Aminocarbonyl-cyclopentyl)phenyl)-4-methoxy-benzamid
N-(4-(1-Aminomethyl-cyclopentyl)phenyl)-4-methoxy-benzamid

Beispiel 1

4-(1,1-Dimethylethyl)-N-(4-methoxyphenyl)benzamid

Zu einer Lösung von 2.50 g (0.02 mol) 4-Methoxyanilin und 3 ml (0.22 mol) Triethylamin in 30 ml Dichlormethan tropfte man unter Eiskühlung und Feuchtigkeitsausschluß innerhalb 1 Stunde eine Lösung von 4.3 ml (0.22 mol) 4-(1,1-Dimethylethyl)-benzoesäurechlorid in 40 ml Dichlormethan. Nach 30 min Rühren bei Raumtemperatur wurde das Lösungsmittel i. Vak. entfernt, der Rückstand mit Wasser digeriert und abgesaugt. Man erhielt 4.2 g Rohprodukt, daß man aus 150 ml Essigester umkristallisierte und bei 40 ° C i. Vak. trocknete:
3.0 g (53 %) farblose Kristalle mit dem Fp. 119-120 ° C.

Analog dem Beispiel 1 erhielt man durch Umsetzung der angegebenen Amine mit 4-(1,1-Dimethylethyl)benzoesäurechlorid die folgenden Verbindungen:

| | Bezeichnung | Ausb. % | Fp °C | umkristall. aus: |
|---|---|---|---|---|
| 2 | 4-(1,1-Dimethylethyl)-N-(4-hydroxyphenyl)benzamid aus 4-Hydroxyanilin | 70 | 197-201 | Toluol |
| 3 | 4-(1,1-Dimethylethyl)-N-(4-propyloxyphenyl)benzamid aus 4-Propyloxyanilin | 62 | 130-132 | Ligroin |
| 4 | N-(4-Allyloxyphenyl)-4-(1,1-dimethylethyl)benzamid aus 4-Allyloxyanilin | 44 | 115-117 | Ethanol |
| 5 | N-(4-Benzyloxyphenyl)-4-(1,1-dimethylethyl)benzamid aus 4-Benzyloxyanilin | 75 | 178-180 | Toluol |
| 6 | 4-(1,1-Dimethylethyl)-N-(4-methylsulfonyloxyphenyl)-benzamid aus 4-Methylsulfonyloxyanilin | | | |
| 7 | 4-(1,1-Dimethylethyl)-N-(2-methoxyphenyl)benzamid aus 2-Methoxyanilin | 45 | Öl | |
| 8 | 4-(1,1-Dimethylethyl)-N-(3-methoxyphenyl)benzamid aus Methoxyanilin | 57 | 105-106 | Ethanol |
| 9 | N-(2-Chlorphenyl)-4-(1,1-dimethylethyl)benzamid aus 2-Chloranilin | 53 | 65-66 | Ligroin |
| 10 | N-(3-Chlorphenyl)-4-(1,1-dimethylethyl)benzamid aus 3-Chloranilin | 87 | 105-107 | Ligroin |

| | Bezeichnung | Ausb. % | Fp °C | umkristall. aus: |
|---|---|---|---|---|
| 11 | N-(4-Chlorphenyl)-4-(1,1-dimethylethyl)benzamid aus 4-Chloranilin | 82 | 198-201 | Ethanol |
| 12 | 4-(1,1-Dimethylethyl)-N-(4-methylmercapto-phenyl)benzamid aus 4-Methylmercaptoanilin | 60 | 119-121 | Toluol |
| 13 | 4-(1,1-Dimethylethyl)-N-(4-methylphenyl)benzamid aus 4-Methylanilin | 70 | 123-125 | Ligroin |
| 14 | N-(4-Cyanophenyl)-4-(1,1-dimethylethyl)benzamid aus 4-Cyanoanilin | 70 | 188-190 | Ethanol |
| 15 | 4-(1,1-Dimethylethyl)-N-(4-nitrophenyl)benzamid aus 4-Nitroanilin | 68 | 158-159 | Ethanol |
| 16 | N-(4-Dimethylaminophenyl)-4-(1,1-dimethylethyl)benzamid aus 2-Methoxyanilin | 49 | 148-150 | Isohexan |
| 17 | N-(3,4-Dimethoxyphenyl)-4-(1,1-dimethylethyl)benzamid aus 3,4-Dimethoxyanilin | 55 | 266-268 | Ethanol |
| 18 | 4-(1,1-Dimethylethyl)-N-(3,-4-methylendioxyphenyl)benzamid aus 3,4-Methylendioxyanilin | 72 | 128-129 | Ligroin |
| 19 | 4-(1,1-Dimethylethyl)-N-(4-(3-pyridinyloxy)phenyl)benzamid aus 4-(3-Pyridinyloxy)anilin | 63 | 145 | Toluol/ Ligroin |

Beispiel 20

N-(4-Aminophenyl)-4-(1,1-dimethylethyl)benzamid

4.5 g (0.015 mol) 4-(1,1-Dimethylethyl)-N-(4-nitrophenyl)benzamid (Beispiel 15) hydrierte man bei Normaldruck und Raumtemperatur in Gegenwart von 0.5 g 10 % Palladium auf Kohle. Nach 30 min wurde filtriert und das Filtrat i.Vak. eingeengt. Der Rückstand wurde mit Ligroin digeriert und abgesaugt. Man

erhielt 3.4 g (85 %) der Titelverbindung mit dem Fp. 111-113 °C.

Beispiel 21

N-(4-Methoxyphenyl)-4-(1-cyanoethyl)benzamid

Analog dem Beispiel 1 setzte man 0.17 g 4-Methoxyanilin mit 0.3 g 4-(1-Cyanoethyl)benzoesäurechlorid um und erhielt nach Umkristallisation aus Methanol 0.23 g (60 %) der Titelverbindung als cremefarbenes Pulver mit dem Fp. 176-178 °C.

4-(1-Cyanoethyl)benzoesäurechlorid stellte man wie folgt her:
1.75 g (10 mmol) 4-(Cyanomethyl)benzoesäuremethylester (J.F. Codington et al., J.Org.Chem. 17 (1952) 1035) und 0.28 g Tetrabutylammoniumbromid in 9.7 ml 50 % Natronlauge versetzte man unter Eiskühlung mit 0.62 ml Iodmethan, gab nach 1 h Dichlormethan und Eiswasser zu, trennte das Dichlormethan ab, wusch es mit Wasser neutral, trocknete und entfernte das Lösungsmittel i.Vak. Das zurückbleibende Öl reinigte man säulenchromatographisch (Kieselgel, n-Heptan/Methylethylketon = 6:1). Die entsprechenden Fraktionen wurden vereinigt und das Lösungsmittel i.Vak. entfernte. Man erhielt 0.57 g (30 %) 4-(1-Cyanoethyl)benzoesäuremethylester als Öl.

Diesen Ester rührte man 4 h in 5 ml Methanol und 1.7 ml 2 N Natronlauge, entfernte das Lösungsmittel i.Vak., säuerte mit 2 N HCl an, saugte den Niederschlag ab, wusch mit Wasser und trocknete. Man erhielt 0.32 g (69 %) 4-(1-Cyanoethyl)benzoesäure mit dem Fp. 113-116 °C.

Diese Säure erhitzte man mit 0.37 ml Thionylchlorid auf 80 °C. Nach 15 min entfernte man das Thionylchlorid i. Vak. und erhielt 0.3 g (90 %) 4-(1-Cyanoethyl)benzoesäurechlorid als Öl, das man sofort ohne weitere Reinigung umsetzte.

Beispiel 22

N-(4-Methoxyphenyl)-4-(1-cyano-1-methylethyl)benzamid

Analog dem Beispiel 1 erhielt man durch Umsetzung von 1 g 4-Methoxyanilin mit 1.86 g 4-(1-Cyano-1-methylethyl)benzoesäurechlorid 1.64 g (69 %) der Titelverbindung als cremefarbenes Pulver mit dem Fp. 164-168 °C nach Umkristallisation aus Methanol.

4-(1-Cyano-1-methylethyl)benzoesäurechlorid stellte man wie folgt her:
0.9 g 4-(Cyanomethyl)benzoesäuremethylester (J.F. Codington et al., J.Org.Chem. 17 (1952) 1035) und 0.15 g Tetrabutylammoniumbromid in 5 ml 50 % Natronlauge versetzte man unter Eiskühlung mit 1 ml Iodmethan. Man rührte 16 h bei Raumtemperatur und arbeitet, wie in Beispiel 21 beschriebenen, auf. Man erhielt 1 g (96 %) 4-(1-Cyano-1-methylethyl)benzoesäuremethylester mit dem Fp. 45-50 °C.

0.5 g dieses Esters verseifte man wie in Beispiel 21 beschrieben und erhielt 0.35 g (76 %) 4-(1-Cyano-1-methylethyl)benzoesäure mit dem Fp. 172-178 °C.

Die Säure versetzte man mit Thionylchlorid wie in Beispiel 21 beschrieben und erhielt 4-(1-Cyano-1-methylethyl)benzoesäurechlorid als Öl, welches man ohne weitere Reinigung einsetzte.

Beispiel 23

N-(4-Methoxyphenyl)-4-(1-aminocarbonyl-1-methylethyl)benzamid

1 g N-(4-Methoxyphenyl)-4-(1-cyano-1-methylethyl)benzamid (Beispiel 22) in 6 ml Dichlormethan versetzte man unter Eiskühlung und kräftigem Rühren mit 1.59 ml $H_2O_2$ (30 %), 0.23 g Tetrabutylammoniumhydrogensulfat und 1.27 ml 20 % Natronlauge.

Man rührte 8 h bei Raumtemperatur, trennte die organische Phase ab, entfernte das Lösungsmittel i.Vak. und reinigte den kristallinen Rückstand säulenchromatographisch. (Kieselgel, n-Heptan/Methylethylketon 1:1). Entsprechende Fraktionen wurden i.Vak. eingeengt. Man erhielt 0.16 g (15 %) der Titelverbindung mit dem Fp. 176-180 °C.

Beispiel 24

N-(4-(1,1-Dimethylethyl)phenyl)-4-methoxy-zimtsäureamid

Unter den Reaktionsbedingungen von Beispiel 1 setzte man 4-(1,1-Dimethylethyl)anilin (2.5 g) mit 4-Methoxyzimtsäurechlorid (2.5 g) um und erhielt 1.0 g der Titelverbindung mit dem Fp. 158-160 °C.

Beispiel 25

4-(1,1-Dimethylethyl)-N-(4-methoxy-2-nitrophenyl)benzamid

Zu 16.8 g (0.1 mol) 4-Methoxy-2-nitroanilin in 150 ml wasserfreiem Pyridin tropfte man unter Kühlung mit Eiswasser 19.7 g (0.1 mol) 4-tert.Butylbenzoesäurechlorid. Man rührte noch 1 h bei Raumtemperatur, goß auf 1 L kaltes Wasser und saugte den Niederschlag ab. Man erhielt 32.3 g (98 %) der Titelverbindung mit dem Fp. 103-107 °C in Form gelber Kristalle.

Beispiel 26

4-(2-Amino-1,1-dimethyl-ethyl)-N-(4-methoxyphenyl)benzamid

2 g 4-(1-Cyano-1-methyl-ethyl)-N-(4-methoxyphenyl)benzamid (Beispiel 22) in 100 ml 10 % ethanolischem Ammoniak hydrierte man in Gegenwart von 1 g 5 % Rhodium auf Aluminiumoxid 24 h bei 5 bar. Man saugte ab, engte das Filtrat zur Trockene ein und kristallisierte den Rückstand aus Ethanol um. Man erhielt 0.68 g (34 %) der Titelverbindung als farblose Kristalle mit dem Fp. 139-140 °C.

Beispiel 27

N-[4-(1-Cyano-1-methylethyl)phenyl]-4-methoxy-benzamid

Analog dem Beispiel 25 setzte man 4-(1-Cyano-1-methylethyl)anilin mit 4-Methoxybenzoesäurechlorid um und erhielt in 85 % Ausbeute die Titelverbindung mit dem Fp. 169-171 °C als beige Kristalle. Eine Probe davon kristallisierte man aus Ethanol um und erhielt einen Fp. von 172-174 °C.

Beispiel 28

N-[4-(1-Aminocarbonyl-1-methylethyl)phenyl]-4-methoxy-benzamid

5 g N-[4-(1-Cyano-1-methylethyl)phenyl]-4-methoxy-benzamid (Beispiel 27) rührte man 20 h bei Raumtemperatur in 100 ml konz. Schwefelsäure, goß auf Eis, saugte den Niederschlag ab, löste ihn in heißem Eisessig, gab Wasser bis zur beginnenden Trübung zu und ließ abkühlen. Man erhielt 2.2 g (42 %) der Titelverbindung als farblose Kristalle mit dem Fp. 234-235 °C.

Beispiel 29

N-[4-(2-Amino-1,1-dimethylethyl)phenyl]-4-methoxy-benzamid

5 g N-[4-(1-Cyano-1-methylethyl)phenyl]-4-methoxy-benzamid (Beispiel 27) hydrierte man bei 90 °C und 120 bar Wasserstoffdruck in 40 ml Ethanol in Gegenwart von 40 ml flüssigem Ammoniak und 2 g Raney-Nickel. Man filtrierte und engte das Filtrat im Vak. zur Trockene ein. nach säulenchromatographischer Reinigung (400 ml Kieselgel 60, Dichlormethan/methanol. Ammoniak = 30:1) erhielt man 2.6 g farblose Kristalle, die man in 100 ml 1 N Salzsäure erhitzte. Nach dem Abkühlen erhielt man 2.3 g (40 %) der Titelverbindung als Hydrochlorid mit dem Fp. 273-276 °C als farblose Blättchen.

Beispiel 30

N-[4-(1,1-Dimethylethyl)phenyl]-4-methoxy-benzamid

Analog dem Beispiel 1 erhielt man aus 4-(1,1-Dimethylethyl)anilin und 4-Methoxybenzoesäurechlorid die Titelverbindung in 65 % Ausbeute mit dem Fp. 167-168 °C.

Beispiel 31

4-(1,1-Dimethylethyl)-N-[4-(ethoxycarbonylmethyloxy)phenyl]benzamid

Zu einer Lösung von 0.66 g Natrium in 25 ml Ethanol gab man 7 g 4-(1,1-Dimethylethyl)-N-(4-hydroxyphenyl)benzamid (Beispiel 2) und 3.2 ml Bromessigsäureethylester. Man erhitzte 10 h unter Rückfluß zum Sieden, entfernte das Lösungsmittel i. Vak. und kristallisierte aus Toluol um. Man erhielt 5.5 g (60 %) der Titelverbindung mit dem Fp. 119-122 °C.

Beispiel 32

4-(1,1-Dimethylethyl)-N-[4-(hydroxycarbonylmethyloxy)phenyl]benzamid

2.0 g 4-(1,1-Dimethylethyl)-N-[4-(ethoxycarbonyl-methyloxy)phenyl]benzamid (Beispiel 31) erhitzte man in 20 ml 2 N Natronlauge, säuerte mit konz. Salzsäure an, saugte ab und wusch mit Wasser nach. Nach Umkristallisieren aus Essigester erhielt man 1.2 g (65 %) der Titelverbindung mit dem Fp. 179-181 °C.

Beispiel 33

4-(1,1-Dimethylethyl)-N-[4-(3-(ethoxycarbonyl)propyloxy)phenyl]benzamid

analog dem Beispiel 31 erhielt man durch Umsetzung von 4-Brombuttersäureethylestermit 4-(1,1-Dimethylethyl)-N-(4-hydroxyphenyl)benzamid (Beispiel 2) die Titelverbindung in 56 % Ausbeute mit dem Fp. 100-101 °C nach Umkristallisation aus Isopropanol.

Beispiel 34

4-(1,1-Dimethylethyl)-N-[4-(3-(hydroxycarbonyl)propyloxy)phenyl]benzamid

Analog dem Beispiel 32 verseifte man die Verbindung des Beispiels 33 und erhielt die Titelverbindung mit dem Fp. 162-168 °C in 67 % Ausbeute nach Umkristallisation aus Essigester.

Beispiel 35

4-(1,1-Dimethylethyl)-N-[4-(((N′-cyclohexyl-N′-methyl-amino)carbonyl)propyloxy)phenyl]benzamid

1.7 g der in Beispiel 34 erhaltenen Säure und 10 ml Thionylchlorid erhitzte man 1.5 h unter Rückfluß zum Sieden. Man entfernte das Thionylchlorid i.Vak. versetzte den Rückstand mit 15 ml Dichlormethan und 1 ml N-Cyclohexyl-N-methylamin und rührte 2 h bei Raumtemperatur. Man schüttelte mit Wasser, dampfte die organische Phase zur Trockene ein und reinigte den Rückstand säulenchromatographisch (Dichlormethan/Methanol 98:2). Nach Eindampfen der gewünschten Fraktionen und Umkristallisation des Rückstandes aus Essigester erhielt man 0.5 g (26 %) der Titelverbindung mit dem Fp. 110-113 °C.

Beispiel 36

4-(1,1-Dimethylethyl)-N-[4-(hydroxyethyloxy)phenyl]benzamid

2.2 g der Verbindung aus Beispiel 31 reduzierte man mit 0.65 g Lithiumaluminiumhydrid in 50 ml siedendem Diethylether. Man versetzte mit Wasser, gab soviel 10 % Schwefelsäure zu, daß sich der Niederschlag gerade löste, extrahierte mit Ether, dampfte zur Trockene ein und reinigte das zurückbleibende Öl säulenchromatographisch (Kieselgel, Dichlormethan, Methanol 99:1). Entsprechende Fraktionen engte

man im Vak. zur Trockene ein, kristallisierte den Rückstand zuerst in Essigester/Ligroin (1:1) und dann mit Toluol um und erhielt so 0.6 g (31 %) der Titelverbindung mit dem Fp. 136-142 °C.

Beispiel 37

4-(1,1-Dimethylethyl)-N-[(4-methoxyphenyl)methyl]benzamid

Analog dem Beispiel 1 erhielt man aus 4-(1,1-Dimethylethyl)benzoesäurechlorid und 4-(Methoxyphenyl)methylamin die Titelverbindung in 71 % Ausbeute mit dem Fp. 145-148 °C nach Umkristallisation aus Isohexan.

Beispiel 38

4-(1,1-Dimethylethyl)-N-[2-nitro-4-(3-pyridinyloxy)phenyl]benzamid

Zu 2.35 g 4-(1,1-Dimethylethyl)-N-[4-(3-pyridinyloxy)phenyl)benzamid in 60 ml Essigsäureanhydrid tropfte man innerhalb 2 h 2.7 ml 100 % $HNO_3$ (d = 1.52), goß auf Eis, extrahierte mit Dichlormethan, entfernte das Lösungsmittel i. Vak. und kristallisierte aus Toluol/Ligroin um. Man erhielt 0.82 g (31 %) der Titelverbindung mit dem Fp. 103 °C.

Beispiel 39

4-(1-Cyanocyclopentyl)-N-(4-methoxyphenyl)-benzamid

a) Zu 22.3 g (127 mmol) 4-(Cyanomethyl)-benzoesäuremethylester (J.F. Codington, E. Mosettig; J.Org.Chem. 17 (1952) 1032) und 1.1 g Benzyltributylammoniumbromid in 87 ml konz. Natronlauge tropfte man rasch 16.5 ml (140 mmol) 1,4-Dibrombutan. Die Temperatur steig auf 70 °C. Man rührte 2 h, wobei die Lösung auf Raumtemperatur abkühlte. Man gab 500 ml Eiswasser zu und extrahierte mit Dichlormethan. Die organische Phase schüttelte man mit Wasser aus, trocknete sie über Natriumsulfat, filtrierte und engte i. Vak. zur Trockene ein. Den Rückstand filtrierte man mit Dichlormethan über eine Säule (L = 50 cm, Ø = 5 cm, Kieselgel "60"). Nach Entfernen des Lösungsmittels i. Vak. erhielt man 21 g (72 %) 4-(1-Cyanocyclopentyl)-benzoesäuremethylester mit Fp. 32-33 °C.

b) 19.4 g (85 mmol) 4-(1-Cyanocyclopenyl)benzoesäuremethylester bewahrte man 20 h bei Raumtemperatur in 180 ml Methanol und 60 ml 2 N Natronlauge auf. Man entfernte das Lösungsmittel i. Vak., säuerte mit 6 N Salzsäure an, filtrierte und wusch das Filtrat mit Wasser. Man erhielt 18 g 4-(1-Cyanocyclopentyl)-benzoesäure (98 %) mit den Fp. 170-174 °C.

c) 5.8 g (27 mmol) 4-(1-Cyanocyclopentyl)benzoesäure setzte man mit Thionylchlorid und anschließend mit 4-Methoxyanilin um wie in Beispiel 1 beschrieben, und erhielt 5.1 g (59 %) der Titelverbindung mit dem Fp. 163-168 °C.

Beispiel 40

4-(1,1-Dimethylethyl)-N-(4-(2-hydroxyethyloxy)phenyl)-benzamid

2.2 g (6.2 mmol) der in Beispiel 31 hergestellten Titelverbindung in 50 ml trockenem Ether erhitzte man mit 1 g $LiAlH_4$, gab anschließend Wasser und wenig 10 % $H_2SO_4$ zu, extrahierte mit Ether, trocknete über $Na_2SO_4$, filtrierte, entfernte das Lösungsmittel i. Vak. und kristallisierte den Rückstand aus Toluol um. Fp. 136-142 °C. Ausbeute 25 %.

## Patentansprüche

**1.** Arzneimittel, enthaltend neben üblichen Träger- und Hilfsstoffen mindestens eine Verbindung der Formel I

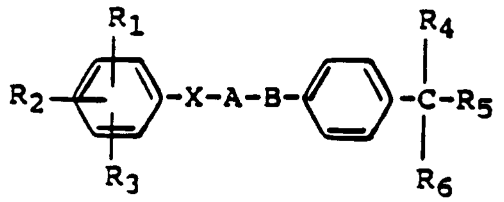

(I)

in der

$R_1$, $R_2$, $R_3$, gleich oder verschieden sein können und jeweils ein Wasserstoffatom, eine $C_1$-$C_6$-Alkyl-, $C_2$-$C_6$-Alkenyl-, $C_2$-$C_6$-Alkinyl-, $C_3$-$C_7$-Cycloalkyl-, $C_3$-$C_7$-Cycloalkenyl-, Phenyl-, Halogen-, Nitro-, Amino-, Formyl-, Hydroxy-, Mercapto-, Cyanogruppe, eine durch eine $C_1$-$C_6$-Alkyl-, $C_2$-$C_6$-Alkenyl-, $C_2$-$C_6$-Alkinyl-, $C_3$-$C_7$-Cycloalkyl-, Phenyl-, Benzyl-, Pyridinyl-, $C_1$-$C_6$-Alkylsulfonyl-, Trifluormethylsulfonyl-, $C_1$-$C_6$-Alkylcarbonyl-, Cyanalkyl-, Hydroxy-$C_1$-$C_6$-alkyl-, Di-$C_1$-$C_6$-alkylamino-$C_1$-$C_6$-alkyl-, Aminocarbonyl-$C_1$-$C_6$-alkyl-, Di-$C_1$-$C_6$-alkylaminocarbonyl-$C_1$-$C_6$-alkyl-, N-Cycloalkyl-N-$C_1$-$C_6$-alkylaminocarbonyl-$C_1$-$C_6$-alkyl-, Carboxy-$C_1$-$C_6$-alkyl-, $C_1$-$C_6$-Alkoxycarbonyl-$C_1$-$C_6$-alkyl- oder $C_1$-$C_6$-alkoxy-$C_1$-$C_6$-alkylgruppe substituierte Hydroxygruppe, eine durch eine oder zwei Trifluormethylsulfonyl-, $C_1$-$C_6$-Alkylcarbonyl-, Formyl-, Aminocarbonyl-, $C_1$-$C_6$-Alkylaminocarbonyl- oder $C_1$-$C_6$-Alkylgruppen substituierte Aminogruppe, eine durch eine $C_1$-$C_6$-Alkylgruppe substituierte Carbonylgruppe, eine durch eine Amino-, $C_1$-$C_6$-Alkylamino-, Piperidino-, Morpholino- oder Thiomorpholinogruppe substituierte Sulfonylgruppe, eine $C_1$-$C_6$-Alkylthio-, $C_1$-$C_6$-Alkylsulfinyl- oder $C_1$-$C_6$-Alkylsulfonylgruppe bedeuten können, oder in der zwei zueinander ortho-ständige Substituenten $R_2$ und $R_3$ zusammen mit den C-Atomen, an die sie gebunden sind, einen fünf- oder sechsgliedrigen Ring bilden,

X eine Bindung, eine $C_1$-$C_6$-Alkylengruppe, oder für den Fall, daß A die Carbonylgruppe -CO- bedeutet, auch die $C_2$-$C_6$-Alkenylengruppe bedeuten kann, und

A und B verschieden sind und die Carbonylgruppe -CO- oder die Aminogruppe -NH- bedeuten, und

$R_4$ eine Methyl-, Cyano-, Aminocarbonyl- oder Aminomethylgruppe bedeutet,

$R_5$ ein Wasserstoffatom, oder eine $C_1$-$C_6$-Alkylgruppe bedeutet,

$R_6$ eine $C_1$-$C_6$-Alkyl- oder $C_3$-$C_7$-Cycloalkylgruppe bedeutet, oder

$R_5$ und $R_6$ zusammen mit dem C-Atom, an das sie gebunden sind, einen $C_3$-$C_7$-Cycloalkylring bedeuten,

deren optisch aktiven Formen, deren Tautomere und deren physiologisch verträglichen Salze anorganischer und organischer Säuren.

**2.** Arzneimittel gemäß Anspruch 1, dadurch gekennzeichnet daß

$R_1$ ein Wasserstoffatom, die Nitro-, Amino-, Fluor-, Chlor-, Brom-, Dimethylamino-, Diethylamino-, Cyano-, Methyl-, Ethyl-, n-Propyl-, i-Propyl-, n-Butyl-, i-Butyl-, tert.-Butyl-, Allyl-, Hydroxy-, Methoxy-, Ethoxy-, Propyloxy-, Allyloxy-, Mercapto-, Methylthio-, Methylsulfinyl-, Methylsulfonyl-, Methylsulfonyloxy-, Cyanmethyloxy-, Benzyloxy-, Pyridinyloxy-, Ethoxycarbonylmethyloxy-, Hydroxymethyl-, Formylamino-, Acetylamino- und die Trifluormethylsulfonylaminogruppe bedeuten kann,

$R_2$ ein Wasserstoffatom, die Methyl-, Ethyl-, n-Propyl-, i-Propyl-, tert.-Butyl-, Chlor-, Methoxy-, Hydroxy- oder Dimethylaminogruppe bedeutet,

$R_3$ ein Wasserstoffatom, die Hydroxy- oder Methoxygruppe bedeutet,

X eine Bindung, die Ethylengruppe oder für den Fall, daß A die Carbonylgruppe bedeutet, auch die Vinylengruppe bedeutet,

A und B verschieden sind und die Amino- und die Carbonylgruppe bedeuten,

$R_4$ die Methyl-, Cyano-, Aminocarbonyl- oder Aminomethylgruppe bedeutet,

$R_5$ ein Wasserstoffatom, die Methyl- oder Ethylgruppe bedeutet,

$R_6$ die Methylgruppe, Ethylgruppe oder Cyclopentylgruppe bedeutet, oder

$R_5$ und $R_6$ zusammen mit dem C-Atom, an das sie gebunden sind, die Cyclopentylgruppe bedeuten.

3. Arzneimittel gemäß Anspruch 1 oder 2, dadurch gekennzeichnet, daß $R_1$ den Substituenten in ortho-Position des Phenylrings darstellt und ein Wasserstoff- oder Chloratom, eine $C_1$-$C_4$-Alkoxy- oder Nitrogruppe bedeutet.

4. Arzneimittel gemäß Anspruch 1, 2 oder 3, dadurch gekennzeichnet, daß $R_2$ den Substituenten in meta-Position des Phenylrings darstellt und ein Wasserstoff- oder Chloratom, oder eine $C_1$-$C_4$-Alkoxygruppe bedeutet.

5. Arzneimittel gemäß einem der Ansprüche 1-4, dadurch gekennzeichnet, daß $R_3$ den Substituenten in para-Position des Phenylrings darstellt, und ein Wasserstoff- oder Halogenatom, eine $C_1$-$C_4$-Alkyl-, $C_2$-$C_4$-Alkenyl-, Nitro-, Amino-, Hydroxy-, Cyano-, $C_1$-$C_4$-Alkoxy-, Benzyloxy-, 3-Pyridinyloxy-, $C_1$-$C_4$-Alkylsulfonyloxy-, Hydroxy-$C_1$-$C_4$-alkyloxy-, Carboxy-$C_1$-$C_4$-alkoxy-, $C_1$-$C_4$-Alkoxycarbonyl-$C_1$-$C_4$-alkoxy-, Di-$C_1$-$C_4$-alkylamino-, oder N-$C_5$-$C_6$-Cycolalkyl-N-$C_1$-$C_4$-alkyl-aminocarbonyl-$C_1$-$C_4$-alkoxygruppe darstellt.

6. Arzneimittel gemäß einem der Ansprüche 1-5, dadurch gekennzeichnet, daß $R_4$ die Methyl- oder Cyanogruppe bedeutet, oder $R_5$ eine $C_1$-$C_4$-Alkylgruppe oder ein Wasserstoffatom darstellt, oder $R_6$ eine $C_1$-$C_6$-Alkylgruppe ist, oder $R_5$ und $R_6$ gemeinsam mit dem C-Atom, an das sie gebunden sind, einen Cyclopentyl- oder Cyclohexylring bilden.

7. Verwendung einer Verbindung gemäß einem der Ansprüche 1 - 6 zur Herstellung von Arzneimitteln zur Behandlung von Herz- und Kreislauferkrankungen.

8. Verbindungen der Formel I

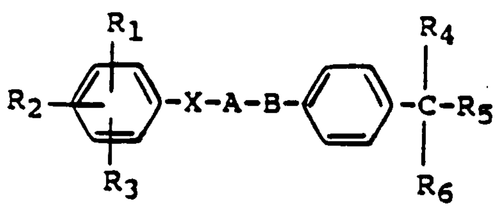

(I)

in der

| | |
|---|---|
| $R_1$ | in ortho-Position des Phenylringes steht und ein Wasserstoffatom oder eine $C_1$-$C_4$-Alkoxy- oder Nitrogruppe bedeutet, |
| $R_2$ | in meta-Position des Phenylringes steht und ein Wasserstoffatom oder eine $C_1$-$C_4$-Alkoxygruppe bedeutet, |
| $R_3$ | in para-Position des Phenylringes steht und eine $C_2$-$C_4$-Alkenyl-, Hydroxy-, Cyano-, $C_1$-$C_4$-Alkoxy-, Benzyloxy-, 3-Pyridinyloxy-, $C_1$-$C_4$-Alkylsulfonyl-oxy-, Hydroxy-$C_1$-$C_4$-alkoxy-, Carboxy-$C_1$-$C_4$-alkoxy-, $C_1$-$C_4$-Alkoxycarbonyl-$C_1$-$C_4$-alkoxy, Di-$C_1$-$C_4$-Alkyl-amino- oder N-$C_5$-$C_6$-Cycloalkyl-N-$C_1$-$C_4$-alkyl-aminocarbonyl-$C_1$-$C_4$-alkoxygruppe bedeutet, |
| X | eine Bindung, eine $C_1$-$C_6$-Alkylengruppe, oder für den Fall, daß A die Carbonylgruppe -CO- bedeutet, auch die $C_2$-$C_6$-Alkenylengruppe bedeuten kann, und |
| A und B | verschieden sind und die Carbonylgruppe -CO- oder die Aminogruppe -NH- bedeuten, und |
| $R_4$ | eine Methyl-, Cyano-, Aminocarbonyl- oder Aminomethylgruppe bedeutet, |
| $R_5$ | ein Wasserstoffatom, oder eine Methylgruppe bedeutet, |
| $R_6$ | eine $C_1$-$C_6$-Alkyl- oder $C_3$-$C_7$-Cycloalkylgruppe bedeutet, oder |
| $R_5$ und $R_6$ | zusammen mit dem C-Atom, an das sie gebunden sind, einen $C_3$-$C_7$-Cycloalkylring bedeuten, |

deren optisch aktiven Formen, deren Tautomere und deren physiologisch verträglichen Salze anorganischer und organischer Säuren,
mit Ausnahme der Verbindung
4-(1,1-Dimethylethyl)-N-[4-(1-methylethoxy)phenyl]-benzamid.

**9.** Verfahren zur Herstellung von Verbindungen der Formel I gemäß Anspruch 8, dadurch gekennzeichnet, daß man in an sich bekannter Weise entweder

a) eine Verbindung der Formel II

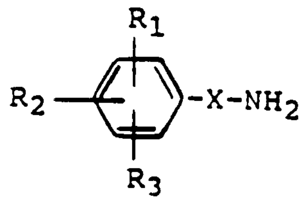

(II)

in der $R_1$, $R_2$ und X die angegebene Bedeutung haben, mit einer Verbindung der Formel III

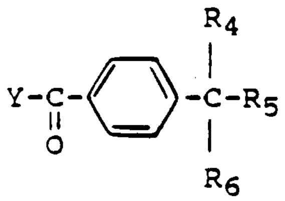

(III)

in der $R_4$, $R_5$ und $R_6$ die angegebene Bedeutung haben, und Y Hydroxy, Halogen, $C_1$-$C_4$-Alkoxy, Phenyloxy, $C_1$-$C_4$-Alkylcarbonyloxy oder Benzoyloxy darstellt, umsetzt, oder

b) eine Verbindung der Formel IV

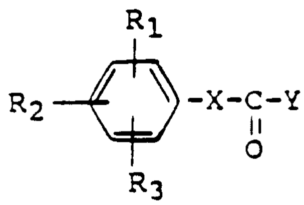

(IV)

in der $R_1$, $R_2$, $R_3$, X und Y die angegebene Bedeutung haben, mit einer Verbindung der Formel V

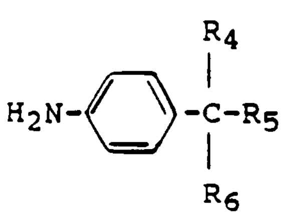

(V)

in der $R_4$, $R_5$ und $R_6$ die angegebene Bedeutung haben, umsetzt,

und anschließend gewünschtenfalls Verbindungen der Formel I in andere Verbindungen der Formel I umwandelt, die Verbindungen in unbedenkliche Salze überführt sowie die optischen Isomeren in

üblicher Weise isoliert.

**10.** Arzneimittel enthaltend mindestens eine Verbindung gemäß Anspruch 8 sowie übliche Träger- und Hilfsstoffe.

**11.** Verwendung von Verbindungen gemäß Anspruch 8 zur Herstellung von Arzneimitteln zur Behandlung von Herz- und Kreislauferkrankungen, insbesondere von Arzneimitteln mit erythrozytenaggregationshemmender Wirkung.

**12.** Verfahren zur Herstellung von Arzneimitteln enthaltend Verbindungen der Formel I gemäß den Ansprüchen 1-7, dadurch gekennzeichnet, daß man eine Verbindung der Formel I zusammen mit physiologisch verträglichen Träger- oder Hilfsstoffen in eine geeignete Darreichungsform bringt.

**Claims**

**1.** Medicaments containing, besides usual carrier and adjuvant materials, at least one compound of the formula I

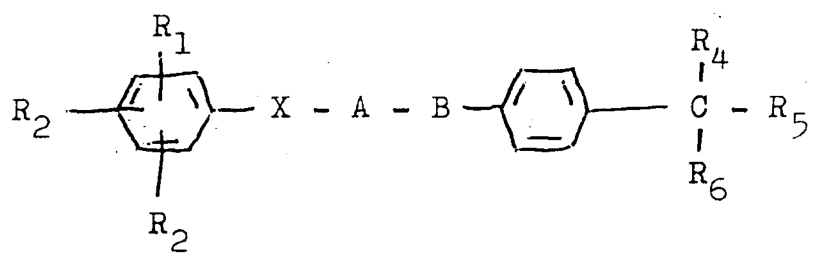

(I)

in which $R_1$, $R_2$, $R_3$ can be the same or different and in each case can signify a hydrogen atom, a $C_1$-$C_6$-alkyl, $C_2$-$C_6$-alkenyl, $C_2$-$C_6$-alkynyl, $C_3$-$C_7$-cycloalkyl, $C_3$-$C_7$-cycloalkenyl, phenyl, halogen, nitro, amino, formyl, hydroxyl, mercapto, cyano group, or a hydroxyl group substituted by a $C_1$-$C_6$-alkyl, $C_2$-$C_6$-alkenyl, $C_2$-$C_6$-alkynyl, $C_3$-$C_7$-cycloalkyl, phenyl, benzyl, pyridinyl, $C_1$-$C_6$-alkylsulphonyl, trifluoromethylsulphonyl, $C_1$-$C_6$-alkylcarbonyl, cyanoalkyl, hydroxy-$C_1$-$C_6$-alkyl, di-$C_1$-$C_6$-alkylamino-$C_1$-$C_6$-alkyl, aminocarbonyl-$C_1$-$C_6$-alkyl, di-$C_1$-$C_6$-alkylaminocarbonyl-$C_1$-$C_6$-alkyl, N-cycloalkyl-N-$C_1$-$C_6$-alkylaminocarbonyl-$C_1$-$C_6$-alkyl, carboxy-$C_1$-$C_6$-alkyl, $C_1$-$C_6$-alkoxycarbonyl-$C_1$-$C_6$-alkyl or $C_1$-$C_6$-alkoxy-$C_1$-$C_6$-alkyl group or an amino group substituted by one or two trifluoromethylsulphonyl, $C_1$-$C_6$-alkylcarbonyl, formyl, aminocarbonyl, $C_1$-$C_6$-alkylaminocarbonyl or $C_1$-$C_6$-alkyl groups, or a carbonyl group substituted by a $C_1$-$C_6$-alkyl group, a sulphonyl group substituted by an amino, $C_1$-$C_6$-alkylamino, piperidino, morpholino or thiomorpholino group, a $C_1$-$C_6$-alkylthio, $C_1$-$C_6$-alkylsulphinyl or $C_1$-$C_6$-alkylsulphonyl group or in which two substituents $R_2$ and $R_3$ standing ortho to one another, together with the C-atoms to which they are attached, form a five- or six-membered ring, X signifies a bond, a $C_1$-$C_6$-alkylene group or, for the case that A signifies the carbonyl group -CO-, can also signify the $C_2$-$C_6$-alkenylene group and A and B are different and signify the carbonyl -CO-group or amino -NH- group and $R_4$ signifies a methyl, cyano, aminocarbonyl or aminomethyl group, $R_5$ signifies a hydrogen atom or a $C_1$-$C_6$-alkyl group, $R_6$ signifies a $C_1$-$C_6$-alkyl or $C_3$-$C_7$-cycloalkyl group or $R_5$ and $R_6$, together with the C-atom to which they are attached, signify a $C_3$-$C_7$-cycloalkyl ring, their optically-active forms, their tautomers and their physiologically compatible salts of inorganic and organic acids

**2.** Medicaments according to claim 1, characterised in that $R_1$ can signify a hydrogen atom, the nitro, amino, fluoro, chloro, bromo, dimethylamino, diethylamino, cyano, methyl, ethyl, n-propyl, i-propyl, n-butyl, i-butyl, tert.-butyl, allyl, hydroxyl, methoxy, ethoxy, propyloxy, allyloxy, mercapto, methylthio, methylsulphinyl, methylsulphonyl, methylsulphonyloxy, cyanomethyloxy, benzyloxy, pyridinyloxy, ethoxycarbonylmethyloxy, hydroxymethyl, formylamino, acetylamino and the trifluoromethylsulphonylamino group, $R_2$ signifies a hydrogen atom, the methyl, ethyl, n-propyl, i-propyl, tert.-butyl, chloro, methoxy, hydroxyl or dimethylamino group, $R_3$ signifies a hydrogen atom, the hydroxyl or methoxy group, X signifies a bond, the ethylene group or, for the case that A signifies the carbonyl group, also signifies the vinylene group, A and B are different and signify the amino and the carbonyl group, $R_4$ signifies the methyl, cyano, aminocarbonyl or aminomethyl group, $R_5$ signifies a hydrogen

atom, the methyl or ethyl group, $R_6$ signifies the methyl group, ethyl group or cyclopentyl group or $R_5$ and $R_6$, together with the C-atom to which they are attached, signify the cyclopentyl group.

**3.** Medicaments according to claim 1 or 2, characterised in that $R_1$ represents the subctituent in orthoposition of the phenyl ring and signifies a hydrogen or chlorine atom, a $C_1$-$C_4$-alkoxy radical or nitro group.

**4.** Medicaments according to claim 1, 2 or 3, characterised in that $R_2$ represents the substituent in meta-position of the phenyl ring and signifies a hydrogen or chlorine atom or a $C_1$-$C_4$-alkoxy group.

**5.** Medicaments according to one of claims 1 - 4, characterised in that $R_3$ represents a substituent in para-position of the phenyl ring and represents a hydrogen or halogen atom, a $C_1$-$C_4$-alkyl, $C_2$-$C_4$-alkenyl, nitro, amino, hydroxyl, cyano, $C_1$-$C_4$-alkoxy, benzyloxy, 3-pyridinyloxy, $C_1$-$C_4$-alkylsulphonyloxy, hydroxy-$C_1$-$C_4$-alkyloxy, carboxy-$C_1$-$C_4$-alkoxy, $C_1$-$C_4$-alkoxycarbonyl-$C_1$-$C_4$-alkoxy, di-$C_1$-$C_4$-alkylamino or N-$C_5$-$C_6$-cycloalkyl-N-$C_1$-$C_4$-alkylaminocarbonyl-$C_1$-$C_4$-alkoxy group.

**6.** Medicaments according to one of claims 1 - 5, characterised in that $R_4$ signifies the methyl or cyano group, or $R_5$ represents a $C_1$-$C_4$-alkyl group or a hydrogen atom or $R_6$ is a $C_1$-$C_6$-alkyl group or $R_5$ and $R_6$, together with the C-atom to which they are attached, form a cyclopentyl or cyclohexyl ring.

**7.** Use of a compound according to one of claims 1 - 6 for the preparation of medicaments for the treatment of heart and circulatory diseases.

**8.** Compounds of the formula I

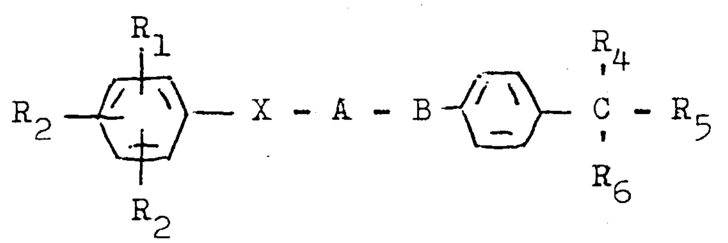

(I)

in which $R_1$ stands in ortho-position of the phenyl ring and signifies a hydrogen atom or a $C_1$-$C_4$-alkoxy or nitro group, $R_2$ stands in meta-position of the phenyl ring and signifies a hydrogen atom or a $C_1$-$C_4$-alkoxy group, $R_3$ stands in para-position of the phenyl ring and signifies a $C_2$-$C_4$-alkenyl, hydroxyl, cyano, $C_1$-$C_4$-alkoxy, benzyloxy, 3-pyridinyloxy, $C_1$-$C_4$-alkylsulphonyloxy, hydroxy-$C_1$-$C_4$-alkoxy, carboxy-$C_1$-$C_4$-alkoxy, $C_1$-$C_4$-alkoxycarbonyl-$C_1$-$C_4$-alkoxy, di-$C_1$-$C_6$-alkylamino or N-$C_5$-$C_6$-cycloalkyl-N-$C_1$-$C_4$-alkylaminocarbonyl-$C_1$-$C_4$-alkoxy group, X signifies a bond, a $C_1$-$C_6$-alkylene group or, for the case that A signifies the carbonyl group -CO-, can also signify a $C_2$-$C_6$-alkenylene group and A and B are different and signify the carbonyl group -CO- or the amino group -NH- and $R_4$ signifies a methyl, cyano, aminocarbonyl or aminomethyl group, $R_5$ signifies a hydrogen atom or a methyl group, $R_6$ signifies a $C_1$-$C_6$-alkyl or $C_3$-$C_7$-cycloalkyl group or $R_5$ and $R_6$, together with the C-atom to which they are attached, signify a $C_3$-$C_7$-cycaloalkyl ring, their optically-active forms, their tautomers and their physiologically compatible salts of inorganic and organic acids, with the exception of the compound 4-(1,1-dimethylethyl)-N-[4-(1-methylethoxy)-phenyl]-benzamide.

**9.** Process for the preparation of compounds of the formula I according to claim 8, characterised in that, in per se known manner, one either

a) reacts a compound of the formula II

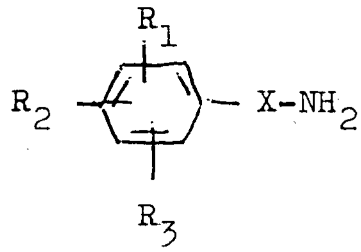

(II)

in which $R_1$, $R_2$ and X have the given meaning, with a compound of the formula III

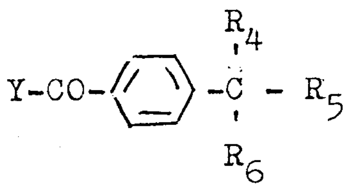

(III)

in which $R_4$, $R_5$ and $R_6$ have the given meaning and Y represents hydroxyl, halogen, $C_1$-$C_4$-alkoxy, phenyloxy, $C_1$-$C_4$-alkylcarbonyloxy or benzyloxy; or
b) reacts a compound of the formula IV

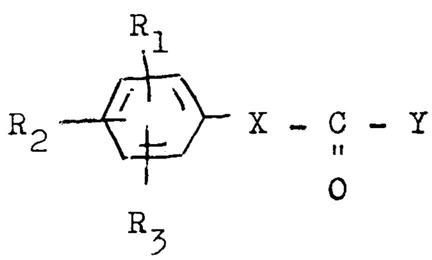

(IV)

in which $R_1$, $R_2$, $R_3$, X and Y have the given meaning, with a compound of the formula V

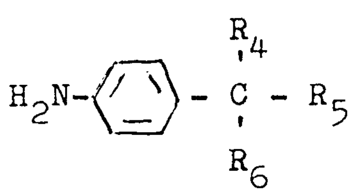

(V)

in which $R_4$, $R_5$ and $R_6$ have the given meaning, and subsequently, if desired, converts compounds of the formula I into other compounds of the formula I, converts the compounds into compatible salts, as well as isolates the optical isomers in usual manner.

**10.** Medicaments containing at least one compound according to claim 8, as well as usual carrier and adjuvant materials.

**11.** Use of compounds according to claim 8 for the preparation of medicaments for the treatment of heart and circulatory diseases, especially medicaments with erythrocyte aggregation-inhibiting action.

**12.** Process for the preparation of medicaments containing compounds of the formula I according to claims 1 - 7, characterised in that one brings a compound of the formula I, together with physiologically compatible carrier and adjuvant materials, into a suitable form of administration.

## Revendications

1. Médicament contenant, en plus de véhicules et d'adjuvants usuels, au moins un composé de formule I

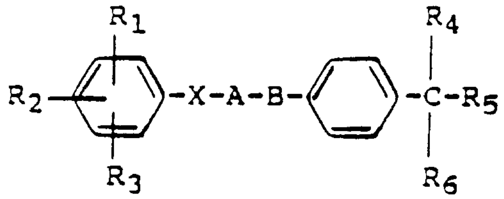

(I)

dans laquelle

- $R_1$, $R_2$, $R_3$ peuvent être identiques ou différents et représentent chacun un atome d'hydrogène, un groupe alkyle($C_1$-$C_6$),alcényle($C_2$-$C_6$), alcynyle($C_2$-$C_6$), cycloalkyle($C_3$-$C_7$), cycloalcényle($C_3$-$C_7$), phényle, halogéno, nitro, amino, formyle, hydroxy, mercapto, cyano ou un groupe hydroxy substitué par un groupe alkyle($C_1$-$C_6$), alcényle($C_2$-$C_6$), alcynyle($C_2$-$C_6$), cycloalkyle($C_3$-$C_7$), phényle, benzyle, pyridinyle, alkylsulfonyle($C_1$-$C_6$), trifluorométhylsulfonyle, alkyl($C_1$-$C_6$)-carbonyle, cyanoalkyle, hydroxyalkyle($C_1$-$C_6$), dialkyl($C_1$-$C_6$)-aminoalkyle($C_1$-$C_6$), aminocarbonylalkyle($C_1$-$C_6$), dialkyl($C_1$-$C_6$)-aminocarbonylalkyle($C_1$-$C_6$), N-cycloalkyl-N-alkyl($C_1$-$C_6$)-aminocarbonylalkyle($C_1$-$C_6$), carboxyalkyle($C_1$-$C_6$), alcoxy($C_1$-$C_6$)-carbonylalkyle($C_1$-$C_6$) ou alcoxy($C_1$-$C_6$)-alkyle-($C_1$-$C_6$), un groupe amino substitué par un ou deux groupes trifluorométhylsulfonyle, alkyl($C_1$-$C_6$)-carbonyle, formyle, aminocarbonyle, alkyl($C_1$-$C_6$)-aminocarbonyle ou alkyle($C_1$-$C_6$), un groupe carbonyle substitué par un groupe alkyle($C_1$-$C_6$), un groupe sulfonyle substitué par un groupe amino, alkyl($C_1$-$C_6$)-amino, pipéridino, morpholino ou thiomorpholino, un groupe alkylthio($C_1$-$C_6$), alkylsulfinyle($C_1$-$C_6$) ou alkylsulfonyle-($C_1$-$C_6$), ou dans laquelle deux substituants $R_2$ et $R_3$, situés en position ortho l'un par rapport à l'autre, forment, conjointement avec l'atome de C auquel ils sont liés, un cycle à cinq ou six chaînons,
- X représente une liaison, un groupe alkylène($C_1$-$C_6$) ou dans le cas où A représente le groupe carbonyle -CO-, il peut également représenter un groupe alcénylène($C_2$-$C_6$), et
- A et B sont différents et représentent un groupe carbonyle -CO- ou un groupe amino -NH-, et
- $R_4$ représente un groupe méthyle, cyano, aminocarbonyle ou aminométhyle,
- $R_5$ représente un atome d'hydrogène ou un groupe alkyle($C_1$-$C_6$),
- $R_6$ représente un groupe alkyle($C_1$-$C_6$), ou cycloalkyle($C_3$-$C_7$), ou
- $R_5$ et $R_6$ forment, conjointement avec l'atome de C auquel ils sont liés, un cycle cycloalkyle-($C_3$-$C_7$),

ses formes optiquement actives, ses tautomères et ses sels physiologiquement acceptables formés avec des acides organiques et inorganiques.

2. Médicament selon la revendication 1, caractérisé en ce que
- $R_1$ représente un atome d'hydrogène, un groupe nitro, amino, fluoro, chloro, bromo, diméthylamino, diéthylamino, cyano, méthyle, éthyle, n-propyle, i-propyle, n-butyle, i-butyle, tert.-butyle, allyle, hydroxy, méthoxy, éthoxy, propyloxy, allyloxy, mercapto, méthylthio, méthylsulfinyle, méthylsulfonyle, méthylsulfonyloxy, cyanométhyloxy, benzyloxy, pyridinyloxy, éthoxycarbonylméthyloxy, hydroxyméthyle, formylamino, acétylamino et trifluorométhylsulfonylamino,
- $R_2$ représente un atome d'hydrogène, un groupe méthyle, éthyle, n-propyle, i-propyle, tert.-butyle, chloro, méthoxy, hydroxy ou diméthylamino,
- $R_3$ représente un atome d'hydrogène, un groupe hydroxy ou méthoxy,
- X représente une liaison, un groupe éthylène ou, dans le cas où A représente un groupe carbonyle, il peut également représenter un groupe vinylène,
- A et B sont différents et représentent un groupe amino ou carbonyle,
- $R_4$ représente un groupe méthyle, cyano, aminocarbonyle ou aminométhyle,

| | |
|---|---|
| $R_5$ | représente un atome d'hydrogène, un groupe méthyle ou éthyle, |
| $R_6$ | représente un groupe méthyle, éthyle ou cyclopentyle, ou |
| $R_5$ et $R_6$ | forment, conjointement avec l'atome de C auquel ils sont liés, un groupe cyclopentyle. |

3. Médicament selon la revendication 1 ou 2, caractérisé en ce que $R_1$ représente les substituants en position ortho du noyau phényle et signifie un atome d'hydrogène ou de chlore, un groupe alcoxy($C_1$-$C_4$) ou nitro.

4. Médicament selon la revendication 1, 2 ou 3, caractérisé en ce que $R_2$ représente les substituants en position méta du noyau phényle et signifie un atome d'hydrogène ou de chlore ou un groupe alcoxy-($C_1$-$C_4$).

5. Médicament selon l'une quelconque des revendications 1-4, caractérisé en ce que $R_3$ représente les substituants en position para du noyau phényle, et signifie un atome d'hydrogène ou d'halogène, un groupe alkyle($C_1$-$C_4$),alcényle($C_2$-$C_4$), nitro, amino, hydroxy, cyano, alcoxy($C_1$-$C_4$), benzyloxy, 3-pyridinyloxy, alkyl($C_1$-$C_4$)-sulfonyloxy, hydroxyalkyloxy($C_1$-$C_4$), carboxyalcoxy($C_1$-$C_4$), alcoxy($C_1$-$C_4$)-carbonyl-alcoxy($C_1$-$C_4$), dialkylamino($C_1$-$C_4$) ou N-cycloalkyle($C_5$-$C_6$), N-alkyl($C_1$-$C_4$)-aminocarbonyl-alcoxy-($C_1$-$C_4$).

6. Médicament selon l'une quelconque des revendications 1-5, caractérisé en ce que $R_4$ représente un groupe méthyle ou cyano, ou $R_5$ représente un groupe alkyle($C_1$-$C_4$) ou un atome d'hydrogène, ou $R_6$ représente un groupe alkyle($C_1$-$C_6$), ou $R_5$ et $R_6$ forment, conjointement avec l'atome de C auquel ils sont liés, un cycle cyclopentyle ou cyclohexyle.

7. Utilisation d'un composé selon l'une quelconque des revendications 1 à 6, pour la préparation de médicaments destinés au traitement de maladies cardiovasculaires.

8. Composés de formule générale I

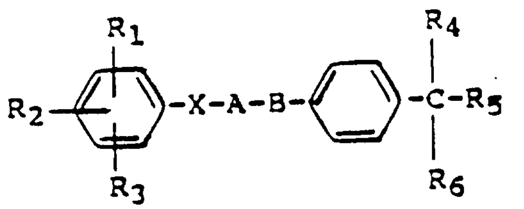

(I)

dans laquelle

| | |
|---|---|
| $R_1$ | est en position ortho du noyau phényle et représente un atome d'hydrogène ou un groupe alcoxy($C_1$-$C_4$) ou nitro, |
| $R_2$ | est en position méta du noyau phényle et représente un atome d'hydrogène ou un groupe alcoxy($C_1$-$C_4$), |
| $R_3$ | est en position para du noyau phényle et représente un groupe alcényle($C_2$-$C_4$), hydroxy, cyano, alcoxy($C_1$-$C_4$), benzyloxy, 3-pyridinyloxy, alkyl($C_1$-$C_4$)-sulfonyloxy, hydroxyalcoxy($C_1$-$C_4$), carboxyalcoxy($C_1$-$C_4$), alcoxy($C_1$-$C_4$)-carbonylalcoxy($C_1$-$C_4$)-, dialkyl($C_1$-$C_4$)-amino ou N-cyloalkyl($C_5$-$C_6$)-N-alkyl($C_1$-$C_4$)-aminocarbonyl-alcoxy($C_1$-$C_4$), |
| X | représente une liaison, un groupe alkylène($C_1$-$C_4$) ou, dans le cas où A représente un groupe carbonyle -CO-, il peut également représenter un groupe alcénylène($C_2$-$C_6$), et |
| A et B | sont différents et représentent un groupe carbonyle -CO- ou un groupe amino -NH-, et |
| $R_4$ | représente un groupe méthyle, cyano, aminocarbonyle ou aminométhyle, |
| $R_5$ | représente un atome d'hydrogène ou un groupe méthyle, |
| $R_6$ | représente un groupe alkyle($C_1$-$C_6$), ou cycloalkyle($C_3$-$C_7$), ou |
| $R_5$ et $R_6$ | forment, conjointement avec l'atome de C auquel ils sont liés, un cycle cycloalkyle($C_3$-$C_7$), |

leurs formes optiquement actives, leurs tautomères et leurs sels physiologiquement acceptables formés

avec des acides organiques et inorganiques,
à l'exception du composé
4-(1,1-diméthyléthyl)-N-[4-(1-méthyléthoxy)phényl]-benzamide.

**9.** Procédé de préparation des composés de formule I selon la revendication 8, caractérisé en ce que, de manière connue en soi,
soit
a) on fait réagir un composé de formule II

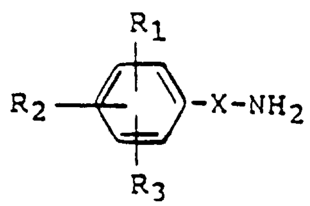

(II)

dans laquelle $R_1$, $R_2$ et X ont les significations mentionnées, avec un composé de formule III

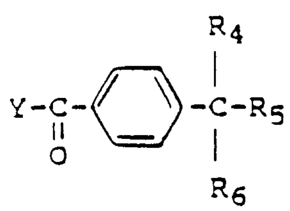

(III)

dans laquelle $R_4$, $R_5$ et $R_6$ ont les significations mentionnées et Y représente un groupe hydroxy, halogéno, alcoxy($C_1$-$C_4$), phényloxy, alkyl($C_1$-$C_4$)-carbonyloxy ou benzoyloxy,
soit
b) on fait réagir un composé de formule IV

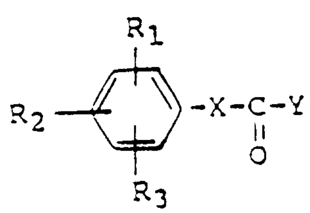

(IV)

dans laquelle $R_1$, $R_2$, $R_3$, X et Y ont les significations mentionnées, avec un composé de formule V

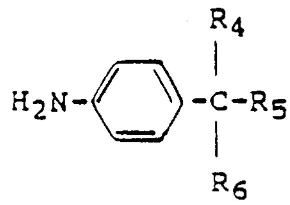

(V)

dans laquelle $R_4$, $R_5$ et $R_6$ ont les significations mentionnées,
et ensuite, si on le souhaite, on transforme les composés de formule I en d'autres composés de formule I, on transforme ces composés en leurs sels physiologiquement acceptables et on isole les isomères optiques de manière usuelle.

**10.** Médicament contenant au moins un composé selon la revendication 8, ainsi que des véhicules et adjuvants usuels.

**11.** Utilisation des composés selon la revendication 8 pour la préparation de médicaments destinés au traitement de maladies cardiovasculaires, en particulier de médicaments ayant une action inhibitrice de l'aggrégation des érythrocytes.

**12.** Procédé de préparation de médicaments contenant des composés de formule I selon les revendications 1-7, caractérisé en ce que l'on transforme un composé de formule I, conjointement avec des véhicules et adjuvants physiologiquement acceptables, en une forme galénique appropriée.